(19) Europäisches Patentamt
European Patent Office
Office européen des brevets

(11) **EP 4 149 921 B1**

(12) **EUROPÄISCHE PATENTSCHRIFT**

(45) Veröffentlichungstag und Bekanntmachung des Hinweises auf die Patenterteilung:
**12.06.2024  Patentblatt 2024/24**

(21) Anmeldenummer: **21726086.8**

(22) Anmeldetag: **12.05.2021**

(51) Internationale Patentklassifikation (IPC):
**C07C 263/10** *(2006.01)*     **C07C 265/14** *(2006.01)*

(52) Gemeinsame Patentklassifikation (CPC):
(C-Sets verfügbar)
**C07C 263/10**                                      (Forts.)

(86) Internationale Anmeldenummer:
**PCT/EP2021/062690**

(87) Internationale Veröffentlichungsnummer:
**WO 2021/228977 (18.11.2021 Gazette 2021/46)**

(54) **VERFAHREN ZUM BETREIBEN EINER ANLAGE ZUR KONTINUIERLICHEN HERSTELLUNG EINES ISOCYANATS**

METHOD FOR OPERATING AN INSTALLATION FOR THE CONTINUOUS PRODUCTION OF AN ISOCYANATE

PROCÉDÉ DE FONCTIONNEMENT D'UNE INSTALLATION DESTINÉE À LA FABRICATION CONTINUE D'UN ISOCYANATE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.05.2020  EP 20174918**

(43) Veröffentlichungstag der Anmeldung:
**22.03.2023  Patentblatt 2023/12**

(73) Patentinhaber: **Covestro Deutschland AG**
**51373 Leverkusen (DE)**

(72) Erfinder:
• **WASTIAN, Dietmar**
**41542 Dormagen (DE)**
• **HIELSCHER, Anke**
**51063 Köln (DE)**
• **SPRIEWALD, Jürgen**
**50769 Köln (DE)**
• **WILDERMUTH, Achim**
**41462 Neuss (DE)**

(74) Vertreter: **Levpat**
**c/o Covestro AG**
**Gebäude K12**
**51365 Leverkusen (DE)**

(56) Entgegenhaltungen:
**WO-A1-2015/144658**

EP 4 149 921 B1

(52) Gemeinsame Patentklassifikation (CPC): (Forts.)

C-Sets
**C07C 263/10, C07C 265/14**

## Beschreibung

Die vorliegende Erfindung betrifft ein Verfahren zum Betreiben einer Anlage zur kontinuierlichen Herstellung eines Isocyanats durch Umsetzung eines primären Amins A mit Phosgen P unter Einhaltung eines auf die Aminogruppen des primären Amins bezogenen stöchiometrischen Überschusses an Phosgen in Gegenwart eines Lösungsmittels L in der Flüssigphase unter Einsatz eines ersten, adiabatisch betriebenen Reaktionsraums und eines zweiten, isotherm betriebenen Reaktionsraums. Das Verfahren zeichnet sich dadurch aus, dass eine Kombination von Maßnahmen, insbesondere die Einhaltung eines hinreichend hohen Startdrucks und einer hinreichend hohen Starttemperatur, angewandt wird, um die Anlage ausgehend von einem Zustand der Produktionsunterbrechung (zum Beispiel nach einem Stillstand wegen Wartungsarbeiten, wegen eines Mangels an Einsatzstoffen oder wegen einer mangelnden Nachfrage nach dem in der Anlage hergestellten Isocyanat) wieder in den Sollzustand, den Regelbetrieb, zu bringen.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen, wobei Phosgen im stöchiometrischen Überschuss eingesetzt wird. Die Umsetzung der Amine mit dem Phosgen kann sowohl in der Gasphase als auch in der Flüssigphase erfolgen, wobei die Reaktion diskontinuierlich oder kontinuierlich durchgeführt werden kann. Die Phosgenierungsreaktion liefert - im Fall der Gasphasenphosgenierung nach der sog. *Quenche* des zunächst anfallenden gasförmigen Reaktionsprodukts - eine Flüssigphase enthaltend das gewünschte Isocyanat.

Verfahren zur Herstellung von organischen Isocyanaten aus primären Aminen und Phosgen sind bereits vielfach beschrieben worden; rein exemplarisch sei auf die folgenden Schriften verwiesen:
DE-A-34 03 204 beschreibt ein kontinuierliches Verfahren zur Herstellung organischer Polyisoyanate, bei welchem bei einem Druck von 5 bis 100 bar in einer zum Teil im Kreislauf geführten Reaktion erhöhte Temperaturen von 100 bis 220 °C eingestellt werden.

DE-A-17 68 439 beschreibt ein Verfahren zur fortlaufenden Herstellung organischer Isocyanate, bei welchem die Einsatzstoffe Amin und Phosgen zunächst vorerhitzt und dann die vorerhitzten Bestandteile in der Reaktionszone unter hohen Drücken zusammengeführt und unter isothermen Bedingungen, d. h. unter Wärmeaustausch mit der Umgebung, zur Reaktion gebracht werden.

DE-A-102 22 968 beschreibt ein Verfahren zur kontinuierlichen Herstellung von Polyisocyanaten durch Umsetzung von primären Aminen mit Phosgen, bei welchem die Umsetzung in einer Kaskade von temperierbaren Reaktionsrohren unterschiedlicher Größe durchgeführt wird.

EP 1 873 142 A1 beschreibt eine dreistufige Verfahrensführung, bei welcher der Druck zwischen der ersten Stufe eines Mischers und der zweiten Stufe eines ersten Phosgenierreaktors gleichbleibt oder ansteigt und in der dritten Stufe, einem Apparat zur Phosgenabtrennung, der Druck niedriger ist als in der zweiten Stufe. Die Reaktion kann adiabatisch oder isotherm betrieben werden.

Von großtechnischem Interesse sind sowohl aromatische Isocyanate wie beispielsweise Methylen-Diphenylen-Diisocyanat (fortan MMDI -"monomeres MDI"), Gemische aus MMDI und Polymethylen-Polyphenylen-Polyisocyanaten (Letztere sind die höheren Homologen des MMDI, fortan als PMDI, "polymeres MDI", bezeichnet) oder Toluylendiisocyanat (TDI) als auch aliphatische Isocyanate wie z. B. 1,5-Pentandiisocyanat (PDI), 1,6-Hexamethylendiisocyanat (HDI), oder Isophorondiisocyanat (IPDI). Daneben sind auch Isocyanate mit benzylischen Isocyanatgruppen bedeutsam, insbesondere sei hier Xylylendiisocyanat (XDI) erwähnt. Die vorliegende Erfindung befasst sich insbesondere mit der Herstellung von Methylen-Diphenylen-Diisocyanaten und Polymethylen-Polyphenylen-Polyisocyanaten (fortan summarisch MDI genannt).

Bei der Mehrzahl der bekannten Verfahren werden zur Einstellung der gewünschten Reaktionstemperatur temperierbare Reaktoren in unterschiedlichen Varianten (Mantelheizung, Beheizung durch Wärmeaustauscher oder spezielle Reaktoreinbauten) eingesetzt. Bei der Isocyanatsynthese durch Phosgenierung von Aminen stellt die externe Temperierung der Reaktoren jedoch oft ein Problem dar, da die hohen Temperaturen der Reaktorwandflächen die Bildung von Nebenprodukten fördern oder sogar erst verursachen, welche dann die Ausbeute und/oder Produkteigenschaften nachteilig beeinflussen. Außerdem werden dann im Reaktor Ablagerungen gebildet, die ein regelmäßiges Abfahren und Reinigen der Reaktoren erforderlich machen. Dies führt aber zum Verlust von Anlagenkapazität und somit zu einem wirtschaftlichen Nachteil. Darüber hinaus verursachen die Wärmeträgeranlagen zusätzliche Investitionskosten, was die Wirtschaftlichkeit des Verfahrens ebenfalls verschlechtert. Zur Lösung dieser Probleme wird in EP 1 616 857 A1 eine zweistufige Verfahrensführung vorgeschlagen, bei welcher in einer ersten Stufe a) Amin und Phosgen in einer *adiabatisch geführten Reaktion* zur Umsetzung gebracht werden, wobei die Temperatur der Umsetzung auf Werte zwischen 100 und 220 °C begrenzt wird, indem der absolute Druck im Reaktor durch Entspannung gezielt auf Werte zwischen 8 und 50 bar eingestellt wird, und die Temperatur bei Werten zwischen 100 und 220 °C so lange beibehalten wird, bis ein Umsatz an Phosgen von mindestens 80 % erreicht ist, und anschließend in einer zweiten Stufe b) das Reaktionsgemisch aus der ersten Stufe auf absolute Drücke im Bereich von 1 bis 15 bar entspannt und bei Temperaturen zwischen 90 und 240°C, *üblicherweise unter Zufuhr von Wärme,* weiter umgesetzt wird. Eine solche Verfahrensführung kann als *adiabatisch-isotherme Verfahrensfüh-*

*rung* bezeichnet werden. Wesentlich für das beschriebene Verfahren ist die Einstellung der Temperatur der Umsetzung im adiabatisch betriebenen Reaktor (100 °C bis 220 °C, bevorzugt 115 °C bis 180 °C, besonders bevorzugt 120 °C bis 150 °C) durch den Druck in diesem Reaktor. Diese Einstellung durch den Druck erfolgt durch kontrolliertes Entspannen mittels Öffnens von an dem Reaktor angebrachten Ventilen, wobei Teile des Reaktionsgemisches aus dem Reaktor entweichen (vgl. Absatz [0016]). Das den adiabatisch betriebenen Reaktor verlassende Reaktionsgemisch wird in einer zweiten Stufe unter isothermen Bedingungen weiter umgesetzt und dabei auf einen Druck unterhalb dem der ersten Stufe entspannt (vgl. Absatz [0019]). Am Ausgang des isotherm betriebenen Reaktors werden diesem eine Gasphase und eine das Isocyanat enthaltende Flüssigphase separat entnommen.

Die europäische Patentanmeldung EP 3 653 605 A1 betrifft ein Verfahren zur Herstellung eines Isocyanats durch Umsetzung eines primären Amins mit Phosgen umfassend **I)** Bereitstellung einer Aminlösung und Temperierung derselben in einem Wärmeaustauscher, **II)** Bereitstellung einer Phosgenlösung und Temperierung derselben in einem Wärmeaustauscher, **III)** Vermischen der Amin- mit der Phosgenlösung in einer Mischeinrichtung gefolgt von **IV)** weiterer Umsetzung in einer adiabatisch betriebenen Reaktionszone und dem Abtrennen der infolge der chemischen Umsetzung gebildeten Gasphase in einer Trennzone, **V)** Entspannen der verbleibenden Flüssigphase, **VI)** weitere Umsetzung der nach Entspannung verbleibenden Flüssigphase in einer indirekt beheizten Reaktionszone und **VII)** Isolierung des Isocyanats aus der dort erhaltenen Reaktionslösung, bei welchem man die Temperatur in der Reaktionszone und Trennzone einstellt, indem man für die Temperatur des Reaktionsgemisches aus Schritt III) einen Soll-Wert innerhalb eines Bereiches von 110 °C bis 145 °C festlegt und die kontinuierlich oder in Intervallen gemessene Ist-Temperatur des Reaktionsgemisches aus Schritt III) zur Regelung der Temperatur der in Schritt I) bereitgestellten Lösung des primären Amins und/oder der Temperatur der in Schritt II) bereitgestellten Lösung von Phosgen mittels der zur Temperierung dieser Lösungen jeweils eingesetzten Wärmeaustauscher verwendet.

Die europäische Patentanmeldung EP 3 653 604 A1 betrifft ein Verfahren zur Herstellung eines Isocyanats durch Umsetzung eines primären Amins mit Phosgen umfassend **I)** Bereitstellung einer Aminlösung, **II)** Bereitstellung einer Phosgenlösung, **III)** Vermischen der Amin- mit der Phosgenlösung in einer Mischeinrichtung gefolgt von **IV)** weiterer Umsetzung in einer adiabatisch betriebenen Reaktionszone und dem Abtrennen der infolge der chemischen Umsetzung gebildeten Gasphase in einer Trennzone, **V)** zwei- bis dreistufiges Entspannen der verbleibenden Flüssigphase, **VI)** weitere Umsetzung der nach der letzten Entspannungsstufe verbleibenden Flüssigphase in einer indirekt beheizten Reaktionszone und **VII)** Isolierung des Isocyanats aus der dort erhaltenen Reaktionslösung.

Die Güte eines Verfahrens zur Herstellung von Isocyanaten ist einerseits definiert durch den Gehalt an unerwünschten Nebenprodukten im Produkt des Verfahrens. Andererseits ist die Güte eines Verfahrens dadurch definiert, dass der gesamte Prozess von Anfahren, Produktion im Regelbetrieb bis zum Abfahren des Prozesses ohne technischen Produktionsausfall und ohne Probleme, die zu einem Eingriff in den Prozess nötigen würden, betrieben werden kann, und dass es nicht zu Verlusten an Einsatzstoffen, Zwischenprodukten oder an Endprodukt kommt. Vor diesem Hintergrund befasst sich die internationale Patentanmeldung WO 2015/144658 A1 mit einem Verfahren zur Herstellung von Isocyanaten durch Phosgenierung der korrespondierenden Amine, bei dem Probleme infolge der Bildung von Ablagerungen in Apparaten der Reaktionsstrecke während der Inbetriebnahme (dem Anfahren) und der Außerbetriebnahme (dem Abfahren) des Verfahrens durch verfahrenstechnische Maßnahmen, insbesondere der Sicherstellung eines sehr großen Überschusses an Phosgen gegenüber dem zu phosgenierenden Amin während der kritischen An- und Abfahrschritte des Verfahrens, vermieden werden. Zu diesem Zweck wird ein kontinuierliches Verfahren zur Herstellung eines Isocyanats durch Umsetzung des korrespondierenden Amins mit Phosgen in einem inerten Lösungsmittel vorgeschlagen, bei welchem nacheinander die Schritte **(A)** Anfahren der kontinuierlichen Produktion, **(B)** kontinuierliche Produktion und **(C)** Abfahren der kontinuierlichen Produktion, durchlaufen werden, wobei in Schritt (A)

eine Mischzone und eine dieser nachgeschaltete Reaktionszone zunächst **(i)** nur mit inertem Lösungsmittel zumindest teilweise beschickt, dann auf die angestrebte Reaktionstemperatur aufgeheizt und danach zusätzlich mit Phosgen, aber nicht mit Amin beschickt werden, oder **(ii)** mit inertem Lösungsmittel und Phosgen ohne das Amin zumindest teilweise beschickt und danach auf die angestrebte Reaktionstemperatur aufgeheizt werden;

erst im Anschluss daran der Reaktionszone das Amin sowie weiteres Phosgen und weiteres inertes Lösungsmittel über die Mischzone kontinuierlich zugeführt werden;

und wobei in Schritt (C) zum Abfahren der kontinuierlichen Produktion zunächst nur die Zufuhr des Amins beendet wird, während Phosgen und inertes Lösungsmittel noch weiter kontinuierlich zugeführt werden. Auf die Besonderheiten einer zweistufigen adiabatisch-isothermen Verfahrensführung geht diese Schrift nicht ein.

Die Erfahrung hat jedoch gelehrt, dass die Erkenntnisse aus "gewöhnlichen" Phosgenierverfahren nicht ohne Weiteres auf die zweistufige adiabatisch-isotherme Verfahrensführung übertragen werden können.

Der vorliegenden Erfindung liegt die Erkenntnis zugrunde, dass in einem zweistufigen adiabatisch-isothermen Phosgenierverfahren mehrere Faktoren gleichzeitig erfüllt sein müssen, damit ein problemloses Anfahren gelingt. Hierzu zählen insbesondere ein *hinreichend hoher Startdruck* und eine *hinreichend hohe (jedoch nicht zu hohe) Starttemperatur.*

Dieser Erkenntnis Rechnung tragend, stellt die vorliegende Erfindung Folgendes bereit:

Ein **Verfahren zum Betreiben einer Anlage zur kontinuierlichen Herstellung eines Isocyanats** durch Umsetzung eines primären Amins A mit Phosgen P unter Einhaltung eines auf die Aminogruppen des primären Amins bezogenen stöchiometrischen Überschusses an Phosgen in Gegenwart eines Lösungsmittels L in der Flüssigphase,

wobei die Anlage folgende Anlagenteile aufweist:

(I) eine erste Mischvorrichtung ausgelegt zur Vermischung von primärem Amin A und Lösungsmittel L zu einer Aminlösung AL,
(II) eine zweite Mischvorrichtung ausgelegt zur Vermischung von Phosgen P und Lösungsmittel L zu einer Phosgenlösung PL,
(III) eine dritte Mischvorrichtung ausgelegt zur Vermischung der Aminlösung und der Phosgenlösung zu einem Reaktionsgemisch,
(IV) einen ersten Reaktionsraum ausgelegt zur adiabatischen Umsetzung des Reaktionsgemisches und einen dem Reaktionsraum nachgeschalteten Entspannungsraum ausgelegt zur Ausbildung einer ersten flüssigen Phase und einer ersten gasförmigen Phase (wobei der Reaktionsraum und der Entspannungsraum entweder (a) in zwei verschiedenen Vorrichtungen, nämlich einem ersten Reaktor und einer ersten Entspannungsvorrichtung, oder (b) in einer gemeinsamen Vorrichtung, die *in ihrer Gesamtheit* einen ersten Reaktor darstellt, angeordnet sind),
(V) eine (im Fall (a): *zweite*) Entspannungsvorrichtung ausgelegt zum Entspannen der ersten flüssigen Phase unter Ausbildung einer zweiten flüssigen Phase und einer zweiten gasförmigen Phase (= Entspannungsraum des Anlagenteils (V), der in einer eigenen Vorrichtung angeordnet ist),
(VI) einen zweiten Reaktionsraum ausgelegt zur isothermen Umsetzung der zweiten flüssigen Phase unter Ausbildung einer dritten flüssigen Phase und einer dritten gasförmigen Phase (= zweiter Reaktor),
(VII) eine Aufarbeitungseinheit zur Gewinnung des Isocyanats aus der dritten flüssigen Phase;

wobei das Verfahren in einem Sollzustand (dem *Re-*

*gelbetrieb*) die kontinuierliche Umsetzung

der Aminlösung in einer Soll-Konzentration an Amin in der Aminlösung von $c(A)_{SOLL}$ und einem Soll-Mengenstrom der Aminlösung von $\dot{m}(AL)_{SOLL}$, welcher sich aus einem Soll-Mengenstrom an Amin von $\dot{m}(A)_{SOLL}$ und einem ersten Soll-Mengenstrom an Lösungsmittel von $\dot{m}(LA)_{SOLL}$ ergibt,

mit der Phosgenlösung in einer Soll-Konzentration an Phosgen in der Phosgenlösung von $c(P)_{SOLL}$ und einem Soll-Mengenstrom der Phosgenlösung von $\dot{m}(PL)_{SOLL}$, welcher sich aus einem Soll-Mengenstrom an Phosgen von $\dot{m}(P)_{SOLL}$ und einem zweiten Soll-Mengenstrom an Lösungsmittel von $\dot{m}(LP)_{SOLL}$ ergibt,

umfasst, und wobei

ausgehend von einem Zustand, in dem der dritten Mischvorrichtung keine Aminlösung zugeführt wird (der momentane Mengenstrom an Aminlösung $\dot{m}(AL)$ also gleich null ist, die Produktion an Isocyanat mithin unterbrochen ist), folgende Schritte zur Erreichung des Sollzustandes durchgeführt werden:

(i) kontinuierliches Zuführen

(a) von Phosgenlösung einer Temperatur im Bereich von 100 °C bis 125 °C, bevorzugt 100 °C bis 105 °C in der Soll-Konzentration $c(P)_{SOLL}$ und dem Soll-Mengenstrom $\dot{m}(PL)_{SOLL}$ aus der zweiten Mischvorrichtung und (gleichzeitig, davor oder danach) (b) von Lösungsmittel (ohne Amin) einer Temperatur im Bereich von 70 °C bis 100 °C, bevorzugt 90 °C bis 95 °C, in dem ersten Soll-Mengenstrom $\dot{m}(LA)_{SOLL}$ aus der ersten Mischvorrichtung

in die dritte Mischvorrichtung und von dort durch den ersten Reaktionsraum, den Entspannungsraum, die Entspannungsvorrichtung und den zweiten Reaktionsraum in die Aufarbeitungseinheit,

(ii) Starten der kontinuierlichen Zufuhr von Amin in die erste Mischvorrichtung, durch welche weiterhin der in Schritt (i)(b) etablierte Strom an Lösungsmittel fließt und sich so eine Aminlösung ausbildet, mit einem Start-Mengenstrom an Amin von $\dot{m}(A)_{START}$, welcher kleiner ist als der Soll-Mengenstrom an Amin $\dot{m}(A)_{SOLL}$, sodass sich eine Temperatur der die erste Mischvorrichtung verlassenden Aminlösung im Bereich von 85 °C bis 105 °C, bevorzugt 95 °C bis 100 °C einstellt und sich eine Temperatur des aus der dritten Mischvorrichtung austretenden Reaktionsgemisches im Bereich von 130 °C bis 145 °C, bevorzugt 138 °C bis 142 °C (**= Starttemperatur**) ergibt, wobei der Zeitpunkt des Startens der kontinuierlichen Zufuhr von Amin A in die erste Mischvorrichtung so gewählt wird, dass beim erstmaligem Aufeinandertreffen von Aminlösung und Phosgenlösung in der dritten Mischvorrichtung ein Druck im Entspannungsraum des Anlagenteils (IV) im Bereich von 16 bar bis 25 bar, bevorzugt 16 bar bis 20 bar (**= Startdruck**) vorliegt,

(iii) Erhöhen des Mengenstroms an Amin von $\dot{m}(A)_{START}$ auf $\dot{m}(A)_{SOLL}$ (in Stufen oder kontinuierlich) und parallel dazu Absenken der Temperatur der aus der zweiten Mischvorrichtung entnommenen Phosgenlösung auf einen Wert im Bereich von 0 °C bis 10 °C, bevorzugt 0 °C bis 5 °C, derart, dass die Temperatur des aus der dritten Mischvorrichtung austretenden Reaktionsgemisches in dem in Schritt ii) eingestellten Bereich bleibt und der Druck im Entspannungsraum des Anlagenteils (IV) auf einen Wert im Bereich von 20 bar bis 30 bar, bevorzugt 20 bar bis 25 bar, ansteigt,

(iv) nach Erreichen des Soll-Mengenstroms an Amin (d. h. $m(A) = \dot{m}(A)_{Soll}$):
Absenken der Temperatur der aus der ersten Mischvorrichtung entnommenen Aminlösung auf einen Wert im Bereich von 50 °C bis 80 °C, bevorzugt 50 °C bis 60 °C, sodass sich im Sollzustand eine Temperatur des aus der dritten Mischvorrichtung austretenden Reaktionsgemisches im Bereich von 125 °C bis 135 °C, bevorzugt 128 °C bis 132 °C, einstellt und der Druck im Entspannungsraum des Anlagenteils (IV) in Bereich von 20 bar bis 30 bar, bevorzugt 20 bar bis 25 bar, bleibt.

Hier und im Folgenden sind alle *Drücke* als absolute Drücke zu verstehen.

Erfindungsgemäß wird Phosgen, bezogen auf die Aminogruppen des primären Amins, im "*stöchiometrischen Überschuss*" eingesetzt. Theoretisch reagiert 1 Mol Phosgen mit 1 Mol primärer Aminogruppen (1 $R-NH_2$ + 1 $COCl_2 \rightarrow$ 1 $R-NCO$ + 2 $HCl$). Ein Phosgenüberschuss von x % gegenüber primären Aminogruppen entspricht daher einem molaren Verhältnis $n(Phosgen)/n(-NH_2)$ ($n$

= Stoffmenge) von $\dfrac{1 + \frac{x}{100}}{1}$, also beispielsweise

$$\frac{1 + \frac{40}{100}}{1} = 1{,}40$$

bei 40%igem Phosgenüberschuss

oder beispielsweise $\dfrac{1 + \frac{120}{100}}{1} = 2{,}2$ bei 120%igem Phosgenüberschuss.

Es folgt zunächst eine Kurzzusammenfassung verschiedener möglicher Ausführungsformen der Erfindung.

In einer **ersten Ausführungsform** der Erfindung, die mit allen anderen Ausführungsformen kombiniert werden kann, werden in Schritt (i)

- die Temperatur der Phosgenlösung auf einen Wert im Bereich von 100 °C bis 105 °C und
- die Temperatur des Lösungsmittels auf einen Wert im Bereich von 90 °C bis 95 °C,

eingestellt;
wobei in Schritt (ii)

- der Druck im Entspannungsraum des Anlagenteils (IV) beim erstmaligem Aufeinandertreffen von Aminlösung und Phosgenlösung in der dritten Mischvorrichtung im Bereich von 16 bar bis 20 bar liegt;
- sich eine Temperatur der die erste Mischvorrichtung verlassenden Aminlösung im Bereich von 95 °C bis 100 °C, einstellt und
- sich eine Temperatur des aus der dritten Mischvorrichtung austretenden Reaktionsgemisches im Bereich von 138 °C bis 142 °C ergibt;

und wobei in Schritt (iii)

- die Temperatur der aus der zweiten Mischvorrichtung entnommenen Phosgenlösung auf einen Wert im Bereich von 0 °C bis 5 °C abgesenkt wird und
- der Druck im Entspannungsraum des Anlagenteils (IV) auf einen Wert im Bereich von 20 bar bis 25 bar ansteigt;

und wobei in Schritt (iv)

- die Temperatur der aus der ersten Mischvorrichtung entnommenen Aminlösung auf einen Wert im Bereich von 50 °C bis 60 °C abgesenkt wird,
- sich im Sollzustand eine Temperatur des aus der dritten Mischvorrichtung austretenden Reaktionsgemisches im Bereich von 128 °C bis 132 °C einstellt und
- der Druck im Entspannungsraum des Anlagenteils (IV) im Bereich von 20 bar bis 25 bar bleibt.

In einer **zweiten Ausführungsform** der Erfindung, die mit allen anderen Ausführungsformen kombiniert werden kann, ist der Druck im Entspannungsraum des Anlagenteils (IV), welcher in Schritt (iii) eingestellt wird, größer als der Druck im Entspannungsraum des Anlagenteils (IV), bei welchem in Schritt (ii) die kontinuierliche Zufuhr von Amin in die erste Mischvorrichtung gestartet wird.

In einer **dritten Ausführungsform** der Erfindung, die eine Alternative zur folgend beschriebenen vierten Ausführungsform ist, ansonsten jedoch mit allen anderen Ausführungsformen kombiniert werden kann, sind der Reaktionsraum und der Entspannungsraum des Anlagenteils (IV) in zwei verschiedenen Vorrichtungen (einem ersten Reaktor und einer ersten Entspannungsvorrichtung) angeordnet.

In einer **vierten Ausführungsform** der Erfindung, die eine Alternative zur zuvor beschriebenen dritten Ausführungsform ist, ansonsten jedoch mit allen anderen Ausführungsformen kombiniert werden kann, sind der Reaktionsraum und der Entspannungsraum des Anlagenteils (IV) in einer gemeinsamen Vorrichtung (die in ihrer Gesamtheit den ersten Reaktor darstellt) angeordnet.

In einer **fünften Ausführungsform** der Erfindung, die mit allen anderen Ausführungsformen kombiniert werden kann, wird in Schritt (ii) der Druck im Entspannungsraum des Anlagenteils (IV) durch ausgasendes Phosgen, durch Zugabe eines Inertgases (insbesondere Stickstoff) und/oder durch Zugabe von Chlorwasserstoff eingestellt.

In einer **sechsten Ausführungsform** der Erfindung, die eine besondere Ausgestaltung der fünften Ausführungsform ist, wird der Druck im Entspannungsraum des Anlagenteils (IV) durch Zugabe von Chlorwasserstoff eingestellt, wobei der Chlorwasserstoff der ersten, zweiten und/oder dritten gasförmigen Phase entnommen wird.

In einer **siebten Ausführungsform** der Erfindung, die eine Alternative zu der folgend beschriebenen achten Ausführungsform und neunten Ausführungsform ist, ansonsten jedoch mit allen anderen Ausführungsformen kombiniert werden kann, wird mit der Durchführung von Schritt (i)(b) **gleichzeitig mit** Schritt (i)(a) begonnen.

In einer **achten Ausführungsform** der Erfindung, die eine Alternative zu der vorstehend beschriebenen siebten Ausführungsform und zur folgend beschriebenen achten Ausführungsform ist, ansonsten jedoch mit allen anderen Ausführungsformen kombiniert werden kann, wird mit der Durchführung von Schritt (i)(b) **vor** Schritt (i)(a) begonnen.

In einer **neunten Ausführungsform** der Erfindung, die eine Alternative zu der vorstehend beschriebenen siebten Ausführungsform und achten Ausführungsform ist, ansonsten jedoch mit allen anderen Ausführungsformen kombiniert werden kann, wird mit der Durchführung von Schritt (i)(b) **nach** Schritt (i)(a) begonnen.

In einer **zehnten Ausführungsform** der Erfindung, die mit allen anderen Ausführungsformen kombiniert werden kann, umfasst die Aufarbeitungseinheit

- eine erste Destillationsvorrichtung ausgelegt zur Abtrennung einer Phosgen-haltigen vierten gasförmigen Phase aus der dritten flüssigen Phase unter Erhalt einer an Phosgen abgereicherten vierten flüssigen Phase,
- eine zweite Destillationsvorrichtung ausgelegt zur Abtrennung einer Lösungsmittelhaltigen fünften gasförmigen Phase aus der vierten flüssigen Phase unter Erhalt einer an Phosgen und Lösungsmittel abgereicherten fünften flüssigen Phase und
- eine dritte Destillationsvorrichtung ausgelegt zur Gewinnung des Isocyanats aus der fünften flüssigen Phase.

In einer **elften Ausführungsform** der Erfindung, die mit allen anderen Ausführungsformen kombiniert werden kann, wird nach Erreichen des Sollzustandes (sowie nach einer Zeit des Betreibens der Anlage im Sollzustand) die kontinuierliche Umsetzung der Aminlösung mit der Phosgenlösung unterbrochen, wobei zur Unterbrechung die folgenden Schritte durchlaufen werden:

(v) Anheben der Temperatur der Phosgenlösung und der Temperatur der Aminösung bei gegenüber dem Sollzustand unveränderten Mengenströmen an Phosgenlösung und Aminlösung;

(vi) bei Erreichen einer Temperatur des aus der dritten Mischvorrichtung austretenden Reaktionsgemisches im Bereich von > 135 °C bis 140 °C: Abstellen der Zufuhr von Amin in die erste Mischvorrichtung unter Beibehaltung der Zufuhr von Lösungsmittel in die erste Mischvorrichtung und unter Beibehaltung der Zufuhr von Phosgenlösung in die zweite Mischvorrichtung;

(vii) Abstellen der Zufuhr von Phosgen in die zweite Mischvorrichtung unter Beibehaltung der Zufuhr von Lösungsmittel in die zweite Mischvorrichtung und unter Beibehaltung der Zufuhr von Lösungsmittel in die erste Mischvorrichtung;

(viii) nach Unterschreiten einer zuvor festgelegten Grenzkonzentration an Phosgen in dem Lösungsmittel, das aus der zweiten Mischvorrichtung austritt: Abstellen der Zufuhr von Lösungsmittel in die zweite Mischvorrichtung; und

(viii) Abstellen der Zufuhr von Lösungsmittel in die erste Mischvorrichtung.

In einer **zwölften Ausführungsform** der Erfindung, die eine besondere Ausgestaltung der elften Ausführungsform ist, wird in Schritt (v) die Temperatur der Phosgenlösung auf einen Wert im Bereich von 100 °C bis 125 °C erhöht, wobei die Temperatur der Aminlösung auf einen Wert im Bereich von 100 °C bis 125 °C erhöht wird.

In einer **dreizehnten Ausführungsform** der Erfindung, die eine besondere Ausgestaltung der elften und zwölften Ausführungsform ist, wird in Schritt (vi) die Aminzufuhr abgeschaltet, sobald die Temperatur des aus der dritten Mischvorrichtung austretenden Reaktionsgemisches im Bereich von > 135 °C bis 137 °C liegt.

In einer **vierzehnten Ausführungsform** der Erfindung, die mit allen anderen Ausführungsformen kombiniert werden kann, ist das primäre Amin ausgewählt aus Methylen-Diphenylen-Diamin (sodass Methylen-Diphenylen-Diisocyanat erhalten wird), Polymethylen-Polyphenylen-Polyamin (sodass Polymethylen-Polyphenylen-Polyisocyanat erhalten wird), einem Gemisch aus Methylen-Diphenylen-Diamin und Polymethylen-Polyphenylen-Polyamin (sodass Gemische aus Methylen-Diphenylen-Diisocyanat und Polymethylen-Polyphenylen-Polyisocyanat erhalten werden), Toluylendiamin (sodass Toluylendiisocyanat erhalten wird), Xylylendiamin (sodass Xylylendiisocyanat erhalten wird), 1,5-Pentandiamin (sodass 1,5-Pentandiisocyanat erhalten wird), 1,6-Hexamethylendiamin (sodass 1,6-Hexamethylendiisocyanat erhalten wird), Isophorondiamin (sodass Isophorondiisocyanat erhalten wird) und Naphthyldiamin (sodass Naphthyldiisocyanat erhalten wird).

Die zuvor kurz geschilderten und weitere mögliche Ausführungsformen der Erfindung werden im Folgenden näher erläutert. Dabei können alle Ausführungsformen beliebig miteinander kombiniert werden, sofern nichts anderes gesagt wird oder sich aus dem Zusammenhang eindeutig ergibt.

BETRIEB DER ANLAGE IM SOLLZUSTAND (REGELBETRIEB)

Bevor auf die Besonderheiten des erfindungsgemäßen Anfahrens der Produktion noch näher eingegangen wird, wird zunächst der Betrieb der Anlage im **Sollzustand** *(= Regelbetrieb)* näher erläutert. Wie für den Fachmann unmittelbar ersichtlich, sind viele der im Folgenden geschilderten Aspekte für den Betriebszustand des Anfahrens gleichermaßen gültig (beispielsweise die Art der eingesetzten Apparaturen oder die Art und Weise der Temperierung der Reaktanden). Auf derartige Aspekte wird zur Vermeidung unnötiger Wiederholungen später nicht mehr näher eingegangen.

Der Sollzustand ist erreicht, sobald der Mengenstrom an Aminlösung m(A) den Wert $\dot{m}(AL)_{SOLL} = \dot{m}(A)_{SOLL} + \dot{m}(LA)_{SOLL}$ (wobei für die Konzentration c(A) an Amin A in der Aminlösung AL $c(A) = c(A)_{SOLL}$ gilt) erreicht. Der Herstellprozess des Isocyanats im Sollzustand umfasst die folgenden Schritte (im Folgenden beschriebene *Verfahrensschritte* mit arabischer Nummerierung ((1), (2), usw.) werden in den *Anlagenteilen* mit entsprechender römischer Nummerierung ((I), (II), usw.) durchgeführt):

(1) Bereitstellen einer Lösung des zu dem Isocyanat korrespondierenden primären Amins A in einem Lösungsmittel L mittels der ersten Mischvorrichtung;

(2) Bereitstellen einer Lösung von Phosgen P in einem Lösungsmittel L mittels der zweiten Mischvorrichtung;

(3) Vermischen der in Schritt (1) bereitgestellten Lösung des primären Amins und der in Schritt (2) bereitgestellten Lösung von Phosgen in der dritten Mischvorrichtung zu einem Reaktionsgemisch einer Temperatur im Bereich von 125 °C bis 135 °C, bevorzugt 128 °C bis 132 °C, unter Einhaltung eines auf die Aminogruppen des primären Amins bezogenen stöchiometrischen Überschusses an Phosgen, bevorzugt im Bereich von 40 % bis 200 % der Theorie, besonders bevorzugt im Bereich von 40 % bis 120 % der Theorie, ganz besonders bevorzugt im Bereich von 50 % bis 100 % der Theorie, und außerordentlich ganz besonders bevorzugt im Bereich von 50 % bis 75 % der Theorie;

(4) Führen des in Schritt (3) erhaltenen flüssigen Reaktionsgemisches durch den ersten Reaktionsraum und durch den dem ersten Reaktionsraum nachgeschalteten Entspannungsraum des Anlagenteils (IV) unter Ausbildung der ersten flüssigen Phase und der unter einem Druck im Bereich 20 bar bis 30 bar, bevorzugt 20 bar bis 25 bar, stehenden ersten gasförmigen Phase, wobei der erste Reaktionsraum und der Entspannungsraum nicht beheizt und nicht gekühlt werden (also beide **adiabatisch** betrieben werden);

(5) (ein- oder mehrstufiges) Entspannen der dem Entspannungsraum aus Schritt (4) entnommenen ersten flüssigen Phase in der Entspannungsvorrichtung des Anlagenteils (V) unter Ausbildung einer zweiten flüssigen Phase und einer zweiten gasförmigen Phase;

(6) Führen der in Schritt (5) nach dem Entspannen erhaltenen zweiten flüssigen Phase durch den zweiten Reaktionsraum unter Ausbildung der dritten (Isocyanat und Lösungsmittel enthaltenden) flüssigen Phase und der dritten (Chlorwasserstoff und Phosgen sowie im Allgemeinen auch noch Anteile verdampften Lösungsmittels enthaltenden) gasförmigen Phase, wobei der zweite Reaktionsraum indirekt beheizt, also **isotherm** betrieben wird;

(7) Aufarbeiten der in Schritt (6) gewonnenen dritten flüssigen Phase in der Aufarbeitungseinheit unter Rückgewinnung des Lösungsmittels und Erhalt des Isocyanats.

Da der Entspannungsraum des Anlagenteils (IV) dem ersten Reaktionsraum *"nachgeschaltet"* ist, womit in der Terminologie der vorliegenden Erfindung eine offene, strömungstechnische Verbindung beider Zonen gemeint ist, und da ferner der erste Reaktionsraum und der Entspannungsraum des Anlagenteils (IV) *"nicht beheizt und nicht gekühlt werden"* (= adiabatische Verfahrensführung), stellt sich an jeder Stelle des ersten Reaktionsraums und des Entspannungsraums des Anlagenteils (IV) eine Temperatur ein, die bei gegebener Temperatur des Reaktionsgemisches aus Schritt (3) im Wesentlichen - abgesehen von Wärmeverlusten durch nicht perfekte Isolierung der verwendeten Apparaturen - von den Reaktionsenthalpien der ablaufenden chemischen Prozesse (die weiter unten noch im Detail erläutert werden) bestimmt wird. Auch der sich etablierende Druck wird zunächst von den ablaufenden chemischen Prozessen bestimmt. Vorzugsweise ist jedoch im Entspannungsraum des Anlagenteils (IV) ein Druckhalteventil für die sich ausbildende Gasphase und eine Flüssigkeitsstandregelung für die Flüssigphase vorgesehen, um sicher gewährleisten zu können, dass Druck im oben genannten Bereich (20 bar bis 30 bar, bevorzugt 20 bar bis 25 bar) liegt. Somit hängt aber die Temperatur in Schritt (4) letztlich von der Temperatur des diesem Schritt zuzuführenden Reaktionsgemisches ab.

**Schritt (1),** die Bereitstellung der für die Phosgenierung erforderlichen Aminlösung, kann nach allen aus dem Stand der Technik bekannten Verfahren erfolgen. Das einzusetzende Amin wird durch das angestrebte Isocyanat bestimmt. Das erfindungsgemäße Verfahren ist grundsätzlich zur Herstellung beliebiger aromatischer, aliphatischer und araliphatischer Isocyanate geeignet. Bevorzugt eingesetzt wird das erfindungsgemäße Verfahren zur Herstellung von Methylen-Diphenylen-Diisocyanat (aus Methylen-Diphenylen-Diamin), Polymethylen-Polyphenylen-Polyisocyanat (aus Polymethylen-Polyphenylen-Polyamin), Gemischen aus Methylen-Diphenylen-Diisocyanat und Polymethylen-Polyphenylen-Polyisocyanat (diese Gemische werden fortan auch als MDI und die Ausgangsamin-Gemische als MDA bezeichnet), Toluylendiisocyanat (aus Toluylendiamin), Xylylendiisocyanat (aus Xylylendiamin), 1,5-Pentandiisocyanat (aus 1,5-Pentandiamin), 1,6-Hexamethylendiisocyanat (aus 1,6-Hexamethylendiamin), Isophorondiisocyanat (aus Isophorondiamin) und Naphthyldiisocyanat (aus Naphthyldiamin), besonders bevorzugt von Methylen-Diphenylen-Diisocyanat, Gemischen aus Methylen-Diphenylen-Diisocyanat und Polymethylen-Polyphenylen-Polyisocyanat sowie Toluylendiisocyanat. Ganz besonders bevorzugt eignet sich das erfindungsgemäße Verfahren zur Herstellung von Methylen-Diphenylen-Diisocyanat und Gemischen aus Methylen-Diphenylen-Diisocyanat und Polymethylen-Polyphenylen-Polyisocyanat. Methylen-Diphenylen-Diisocyanat wird auch als Diamin der Diphenylmethanreihe bezeichnet. Polymethylen-Polyphenylen-Polyisocyanat wird auch als Polyamin der Diphenylmethanreihe bezeichnet.

Verfahren zur Herstellung der genannten Amine sind dem Fachmann bekannt und bedürfen daher keiner weiteren Erläuterung an dieser Stelle.

In Schritt (1) wird das zu phosgenierende Amin in einem Lösungsmittel gelöst. Als hierzu erforderliche erste Mischvorrichtung kann eine dem Fachmann an sich bekannte Mischeinrichtung eingesetzt werden. Insbesondere sind Mischrohre mit statischen Mischern als Einbauten (häufig auch kurz als *Statikmischer* bezeichnet) geeignet. Geeignete erfindungsgemäß einsetzbare *Lösungsmittel* sind dabei unter den Reaktionsbedingungen inerte Lösungsmittel wie z. B. Monochlorbenzol, Dichlorbenzol (insbesondere das *ortho*-Isomer), Dioxan, Toluol, Xylol, Methylenchlorid, Perchlorethylen, Trichlorfluormethan oder Butylacetat. Das Lösungsmittel ist bevorzugt im Wesentlichen frei von Isocyanat (Massenanteil < 100 ppm angestrebt) und im Wesentlichen frei von Phosgen (Massenanteil < 100 ppm angestrebt), worauf bei Einsatz von Recycleströmen zu achten ist. Bevorzugt wird daher nach einem Verfahren, wie in EP 1 854 783 A2 beschrieben, gearbeitet. Die Lösungsmittel können einzeln oder als beliebige Gemische der beispielhaft genannten Lösungsmittel eingesetzt werden. Vorzugsweise werden Monochlorbenzol (MCB) oder *ortho*-Dichlorbenzol (ODB) eingesetzt, ganz besonders bevorzugt Monochlorbenzol (MCB).

Vorgesehen ist eine Temperatur der resultierenden Aminlösung im Bereich von 50 °C bis 80 °C, besonders bevorzugt 50 °C bis 60 °C. Dies kann prinzipiell erreicht werden durch entsprechende Temperierung der Ausgangsstoffe Amin und Lösungsmittel unter Berücksichtigung der Lösungsenthalpie. Es ist erfindungsgemäß jedoch bevorzugt, insbesondere zusätzlich zur genannten Temperierung der Ausgangsstoffe, einen der Vermischung von Amin und Lösungsmittel nachgeschalteten Wärmeaustauscher bereitzustellen, der die gezielte Einstellung der Amin-Lösung auf die gewünschte Temperatur im genannten Bereich ermöglicht, der also je nachdem welche Temperatur unmittelbar nach der Vermischung der Ausgangsstoffe vorliegt, heizen oder kühlen kann. Hierzu eignen sich dem Fachmann bekannte Wärmeaustauscher wie insbesondere Rohrbündelwärmeaustauscher und Plattenwärmeaustauscher.

Im Hinblick auf die Aminkonzentration in der in Schritt (1) bereitgestellten Lösung ist es bevorzugt, den auf die Gesamtmasse dieser Lösung bezogenen Massenanteil an primärem Amin auf einen Wert im Bereich von 25 % bis 50 %, insbesondere im Bereich von 30 % bis 45 %, einzustellen.

**Schritt (2),** die Bereitstellung der für die Phosgenierung erforderlichen Phosgenlösung, kann ebenfalls nach allen aus dem Stand der Technik bekannten Verfahren erfolgen. Es sind die gleichen Mischvorrichtungen und Lösungsmittel geeignet wie zuvor für das primäre Amin beschrieben. Insbesondere ist es bevorzugt, das primäre Amin in Schritt (1) und Phosgen in Schritt (2) jeweils in demselben Lösungsmittel, ganz besonders bevorzugt also in MCB, zu lösen. Verfahren zur Herstellung von Phosgen sind dem Fachmann bekannt und bedürfen daher keiner weiteren Erläuterung an dieser Stelle.

Vorgesehen ist eine Temperatur der resultierenden Phosgenlösung im Bereich 0 °C bis 10 °C, bevorzugt 0 °C bis 5 °C. Dies kann prinzipiell erreicht werden durch entsprechende Temperierung der Ausgangsstoffe Phosgen und Lösungsmittel unter Berücksichtigung der Lösungsenthalpie. Es ist erfindungsgemäß jedoch bevorzugt, insbesondere zusätzlich zur genannten Temperierung der Ausgangsstoffe, einen der Vermischung von Phosgen und Lösungsmittel nachgeschalteten Wärmeaustauscher bereitzustellen, der die gezielte Einstellung der Phosgenlösung auf die gewünschte Temperatur im oben genannten Bereich ermöglicht, der also je nachdem welche Temperatur unmittelbar nach der Vermischung der Ausgangsstoffe vorliegt, heizen oder kühlen kann. Hierzu sind die gleichen Wärmeaustauscher geeignet wie zuvor für das primäre Amin beschrieben.

Im Hinblick auf die Phosgenkonzentration in der in Schritt (2) bereitgestellten Lösung ist es bevorzugt, den auf die Gesamtmasse dieser Lösung bezogenen Massenanteil an Phosgen auf einen Wert im Bereich von 45 % bis 90 %, insbesondere im Bereich von 55 % bis 80 %, einzustellen.

In **Schritt (3)** erfolgt das Vermischen der in Schritt (1) bereitgestellten Lösung des primären Amins und der in Schritt (2) bereitgestellten Lösung des Phosgens. Als hierzu erforderliche dritte Mischvorrichtung eignen sich dem Fachmann an sich bekannte Mischvorrichtungen wie statische oder dynamische Mischer. Statische Mischer sind durch die Abwesenheit beweglicher Teile gekennzeichnet, insbesondere seien hier Mischrohre mit statischen Mischern als Einbauten (häufig auch kurz als *Statikmischer* bezeichnet) oder Düsen genannt. Dynamische Mischer enthalten dagegen bewegliche Teile wie beispielsweise Rührorgane. Insbesondere sind hier auch die aus EP 0 830 894 A1 und EP 2 077 150 A1 bekannten Rotor-Stator-Systeme zu nennen. Dynamische Mischer, insbesondere solche vom Rotor-Stator-Typ, sind für den Einsatz in der vorliegenden Erfindung bevorzugt.

Bevorzugt wird die dritte Mischvorrichtung aus Schritt (3) nicht beheizt und nicht gekühlt, d. h. die Temperatur des erhaltenen Reaktionsgemisches wird allein durch die Mischungsenthalpie und die Enthalpie der bereits in der Mischeinrichtung einsetzenden Reaktionen bestimmt. Eine Transportleitung für das Reaktionsgemisch zwischen dem Austritt der dritten Mischvorrichtung und dem Eintritt in den ersten Reaktionsraum wird vorzugsweise ebenfalls weder beheizt noch gekühlt, ist jedoch vorzugsweise wärmeisoliert.

Erfindungsgemäß wird bei der Vermischung in Schritt (3) ein auf die Aminogruppen des primären Amins bezogener stöchiometrischer Überschuss an Phosgen eingehalten, bevorzugt im Bereich von 40 % bis 200 % der Theorie, besonders bevorzugt im Bereich von 40 % bis 120 % der Theorie, ganz besonders bevorzugt im Bereich von 50 % bis 100 % der Theorie, und außerordentlich ganz besonders bevorzugt im Bereich von 50 % bis 75 % der Theorie.

In **Schritt (4)** findet (abgesehen von bereits in der dritten Mischvorrichtung begonnenen chemischen Umsetzungen) der erste Hauptteil der Reaktion zum Isocyanat statt, und zwar unter adiabatischen Bedingungen. Damit ist gemeint, dass die Schritt (4) durchlaufende Reaktionsmischung während der Umsetzung weder geheizt noch gekühlt wird. Die verwendeten Apparaturen sind gegen Wärmeverluste isoliert, sodass die Temperaturentwicklung von der Reaktionsenthalpie der ablaufenden Umsetzungen bestimmt wird.

Ohne auf eine Theorie festgelegt sein zu wollen ist davon auszugehen, dass in Schritt (4) (und teilweise bereits in Schritt (3)) mehrere Umsetzungen parallel ablaufen. Das primäre Amin reagiert mit Phosgen zur bekannten Zwischenstufe Carbaminsäurechlorid (exotherme Reaktion). Der dabei freigesetzte Chlorwasserstoff reagiert mit noch nicht umgesetztem Amin zu Aminhydrochlorid (exotherme Reaktion), das sich im eingesetzten Lösungsmittel löst (endotherme Reaktion). Partiell findet auch in Schritt (4) bereits die Spaltung des Carbaminsäurechlorids zum gewünschten Isocyanat und Chlorwasserstoff statt (endotherme Reaktion). Die Temperaturänderung hängt vom Zusammenspiel aller dieser Reaktionen statt. In der Regel wird in Schritt (4) nur eine geringe Temperaturänderung beobachtet, was dafür spricht, dass sich exo- und endotherme Reaktionen "die Waage halten". In jedem Fall bildet sich bei den Umsetzungen in Schritt (4) eine erste gasförmige Phase aus, die im ersten Entspannungsraum des Anlagenteils (IV) von der verbleibenden ersten flüssigen Phase getrennt wird.

(a) Es ist erfindungsgemäß möglich, als ersten Reaktionsraum und Entspannungsraum des Anlagenteils (IV) zwei getrennte Vorrichtungen einzusetzen, in welchem Fall in der Terminologie der vorliegenden Erfindung die den ersten Reaktionsraum enthaltende Vorrichtung als *erster Reaktor* und die den ersten Entspannungsraum enthaltende Vorrichtung als *erste Entspannungsvorrichtung* bezeichnet wird. Als erste Entspannungsvorrichtung eignet sich ein dem Fachmann an sich bekannter Gas-Flüssigkeits-Trennbehälter (auch als *Gasabscheider* bezeichnet).

(b) Es ist erfindungsgemäß ebenfalls möglich und bevorzugt, den ersten Reaktionsraum und den ersten Entspannungsraum in einer gemeinsamen Vorrichtung anzuordnen, welche dann in der Terminologie der vorliegenden *Erfindung in ihrer Gesamtheit* als *erster Reaktor* bezeichnet wird.

Unabhängig davon, ob Variante (a) oder Variante (b) verwendet wird, eignen sich als *erster Reaktor* übliche, dem Fachmann bekannte Phosgenierungsreaktoren, wie insbesondere aufrecht angeordnete röhrenförmige Reaktoren (Rohrreaktoren; ist das Verhältnis von Höhe zu Durchmesser relativ klein, spricht man auch von Turmreaktoren oder Reaktortürmen). Solche Reaktoren können auch als Blasensäulen betrieben werden. Zur Einengung der Verweilzeitverteilung kann der im ersten Reaktor angeordnete erste Reaktionsraum durch dem Fachmann bekannte Einbauten segmentiert sein. Der erste Reaktor wird bevorzugt mit dem in Schritt (3) erhaltenen Reaktionsgemisch von unten nach oben durchströmt. Im bevorzugten Fall (b) ist dann der erste Entspannungsraum der Gasraum im oberen Bereich des ersten Reaktors, welchem die erste flüssige Phase und die erste gasförmige Phase getrennt entnommen werden.

Die Phasentrennung findet, unabhängig davon, ob Variante (a) oder (b) verwendet wird, spontan statt.

In **Schritt (5)** wird die in Schritt (4) erhaltene erste flüssige Phase ein- oder mehrstufig entspannt, und zwar bevorzugt auf einen Druck im Bereich von 1,0 bar bis 20 bar, bei mehrstufiger Entspannung gemessen in der in der letzten Stufe erhaltenen Gasphase, welche in der Terminologie der vorliegenden Erfindung als zweite gasförmige Phase bezeichnet wird. Als (im Fall (a): *zweite*) Entspannungsvorrichtung eignen sich dem Fachmann an sich bekannte Gas-Flüssigkeits-Trennbehälter (auch als *Gasabscheider* bezeichnet). Bei mehrstufiger Entspannung in Schritt (5) wird eine entsprechende Anzahl an derartigen Gasabscheidern in Reihe geschaltet, wobei dann in der Terminologie der vorliegenden Erfindung *die Gesamtheit* aller in Reihe geschalteten Gas-Flüssigkeits-Trennbehälter die Entspannungsvorrichtung des Anlagenteils (V) darstellt. Dabei bildet sich in jeder Stufe eine Chlorwasserstoff und nicht umgesetztes Phosgen enthaltende Gasphase aus. In dieser mehrstufigen Entspannung ist die nach Entspannung der ersten Stufe erhaltene Flüssigphase der Einsatzstoff der zweiten Stufe usw.

Es ist möglich, den für die Entspannung (bei mehrstufiger Entspannung den für die *letzte* Entspannung) vorgesehenen Gas-Flüssigkeits-Trennbehälter und den zweiten (indirekt beheizten) Reaktionsraum aus Schritt (6) in einem gemeinsamen Apparat anzuordnen. Ein Beispiel für eine mögliche Ausgestaltung wird unten beschrieben.

In **Schritt (6)** wird die in Schritt (5) erhaltene zweite flüssige Phase im zweiten Reaktionsraum unter Ausbildung der das gewünschte Isocyanat enthaltenden dritten flüssigen Phase und der (Chlorwasserstoff- und Phosgen-haltigen) dritten gasförmigen Phase weiter umgesetzt ("isotherme Verfahrensführung"). Dies kann in dem Fachmann bekannten heizbaren Reaktoren stattfinden. Insbesondere geeignet sind zu diesem Zweck (vertikal angeordnete) Rohrbündelreaktoren. Dabei kann die zweite flüssige Phase durch das Innere der Rohre des Rohrbündelreaktors (Rohrinnenraum) oder durch den Raum zwischen den Rohren des Rohrbündelreaktors, der nach außen durch die das Rohrbündel umhüllende Reaktorwand begrenzt wird (Rohraußenraum), geführt werden. Das Heizmedium - ein Wärmeträgeröl, eine Salzschmelze, Dampf oder dergleichen - wird dann durch den jeweils anderen Raum geführt, sodass es nicht in stofflichen Kontakt mit dem umzusetzenden flüssigen Verfahrensprodukt tritt (indirekte Beheizung). Die Schritt (4) erhaltene zweite flüssige Phase durchläuft dabei den vertikal angeordneten Rohrbündelreaktor vorzugsweise von oben nach unten. In der bevorzugten Ausgestaltung der Erfindung unter Integration des für Entspannung von Schritt (5) bzw. für die letzte Entspannungsstufe von Schritt (5) vorgesehenen Gas-Flüssigkeits-Trennbehälters in den Apparat, in welchem der zweite Reaktionsraum aus Schritt (5) angeordnet ist, findet dann die Gas-Flüssig-Phasentrennung in einer Haube am Kopf des Rohrbündelreaktors statt.

In **Schritt (7),** der Aufarbeitung der in Schritt (6) gewonnenen dritten flüssigen Phase in der Aufarbeitungseinheit wird das gewünschte Isocyanat unter Rückgewinnung des Lösungsmittels isoliert. Dieser Schritt kann nach an sich aus dem Stand der Technik bekannten Verfahren durchgeführt werden und umfasst im Allgemeinen eine Entphosgenierung, eine Lösungsmittelabtrennung und eine Feinreinigung. Bevorzugt wird die dritte flüssige Phase zunächst in einer ersten Destillationsvorrichtung (der sog. "Entphosgenierkolonne") von gelöstem Phosgen (das als vierte gasförmige Phase abgetrennt wird) möglichst vollständig befreit, wobei eine an Phosgen abgereicherte vierte flüssige Phase erhalten wird. Diese wird bevorzugt in einer zweiten Destillationsvorrichtung (der sog. "Lösungsmittelkolonne") von Lösungsmittel (das als fünfte gasförmige Phase abgetrennt wird) möglichst vollständig befreit, wobei eine an Phosgen und Lösungsmittel abgereicherte fünfte flüssige Phase erhalten wird. Zurückgewonnenes Phosgen und Lösungsmittel werden vorteilhafterweise rezykliert.

Die fünfte flüssige Phase wird in einer dritten Destillationsvorrichtung einer Feinreinigung, die erforderlichenfalls eine Homologentrennung umfasst, unterzogen. Abhängig von der Art des Isocyanats kann die Feinreinigung auch eine Isomerentrennung umfassen. In einer bevorzugten Ausführungsform ist die vorliegende Erfindung auf die Herstellung von Methylen-Diphenylen-Diisocyanat und von Gemischen aus Methylen-Diphenylen-Diisocyanat und Polymethylen-Polyphenylen-Polyisocyanat gerichtet. In diesem Fall wird die fünfte flüssige Phase, welche die Gesamtheit des rohen Isocyanat-(Homologen- und Isomeren-)Gemisches enthält, bevorzugt zunächst einer destillativen Homologentrennung zugeführt (der sog. "Polymerabtrennung"), wobei ein Destillat aus Methylen-Diphenylen-Diisocyanat (welches Polymethylen-Polyphenylen-Polyisocyanat allenfalls in Spuren enthält) und ein Sumpfprodukt aus Polymethylen-Polyphenylen-Polyisocyanat und Methylen-Diphenylen-Diisocyanat (in welchem der Anteil an Methylen-Diphenylen-Diisocyanat im Vergleich zur fünften flüssigen Phase entsprechend der in der Homologentrennung abdestillierten Menge an Methylen-Diphenylen-Diisocyanat verringert ist) anfallen. Das Destillat aus Methylen-Diphenylen-Diisocyanat wird in mindestens einem weiteren Destillationsschritt von leicht- und hochsiedenden Verunreinigungen befreit und in verschiedene Isomeren-Fraktionen getrennt. Bevorzugt umfasst die Isomerentrennung die Gewinnung einer Fraktion aus 4,4'-Methylen-Diphenylen-Diisocyanat und einer Fraktion aus einem Gemisch von 2,4'-Methylen-Diphenylen-Diisocyanat und 4,4'-Methylen-Diphenylen-Diisocyanat. Der hier nur kurz umrissene Schritt (7) ist im Stand der Technik ausführlich beschrieben; es sei beispielsweise auf WO 2017/050776 A1 verwiesen.

In den Schritten (4), (5) und (6) fallen gasförmige Phasen (nämlich die erste, zweite bzw. dritte gasförmige Phase) enthaltend Chlorwasserstoff und Phosgen sowie gegebenenfalls mitgerissenes Lösungsmittel an. Bevorzugt werden diese gasförmigen Phasen zur Rückgewinnung von Wertprodukten ebenfalls aufgearbeitet. Die Aufarbeitung dient insbesondere der Trennung von Phosgen und Chlorwasserstoff voneinander und von Verunreinigungen und kann beispielsweise durch Absorption des Phosgens in einem Lösungsmittel oder durch destillative Trennung nach Kompression und Verflüssigung erfolgen. Das erhaltene Chlorwasserstoffgas eignet sich für die weitere Oxidation zu Chlor, welches für die Herstellung des für Schritt (2) erforderlichen Phosgens benötigt wird. Die Oxidation kann elektrolytisch oder katalytisch mit Sauerstoff (sog. *Deacon*-Prozess) erfolgen. Ein Teil des zurückgewonnenen Chlorwasserstoffs kann auch, wie weiter unten noch erläutert wird, zur Einstellung des *Startdrucks* eingesetzt werden. Zurückgewonnenes Phosgen, gegebenenfalls Lösungsmittel enthaltend, kann in Schritt (2) verwendet werden.

In jedem Fall ist es zweckmäßig, die in den Schritten (4), (5) und (6) anfallenden gasförmigen Phasen vor der Aufarbeitung auf einen gemeinsamen Druck einzustellen und zu vereinigen.

Grundsätzlich kann dies geschehen, in dem alle gasförmigen Phasen auf den geringsten Druck (d. h. auf den Druck der in Schritt (6) erhaltenen dritten gasförmigen Phase) oder einen noch geringeren Druck entspannt und dann weiter aufgereinigt werden. Diese Vorgehensweise ist insbesondere bei Aufarbeitung durch Absorption bevorzugt.

ANFAHREN DER PRODUKTION (ÜBERFÜHREN DER ANLAGE IN DEN SOLLZUSTAND)

Für den Anfahrprozess der vorliegenden Erfindung ist es erforderlich, dass, ausgehend von einem Zustand, in dem der dritten Mischvorrichtung keine Aminlösung zugeführt wird (der momentane Mengenstrom an Aminlösung m(AL) also gleich null ist, die Produktion an Isocyanat mithin unterbrochen ist), zur Erreichung des Sollzustandes (zum Überführen der Anlage in den zuvor erläuterten *Regelbetrieb*) die weiter oben beschriebenen **Schritte (i) bis (iv)** durchgeführt werden. In einer bevorzugten Ausführungsform wird dabei

in Schritt (i)

- die Temperatur der Phosgenlösung auf einen Wert im Bereich von 100 °C bis 105 °C und
- die Temperatur des Lösungsmittels auf einen Wert im Bereich von 90 °C bis 95 °C, eingestellt;

wobei in Schritt (ii) so verfahren wird, dass

- der Druck im Entspannungsraum des Anlagenteils (IV) beim erstmaligem Aufeinandertreffen von Aminlösung und Phosgenlösung in der dritten Mischvorrichtung im Bereich von 16 bar bis 20 bar liegt;
- sich eine Temperatur der die erste Mischvorrichtung verlassenden Aminlösung im Bereich von 95 °C bis 100 °C, einstellt und
- sich eine Temperatur des aus der dritten Mischvorrichtung austretenden Reaktionsgemisches im Bereich von 138 °C bis 142 °C ergibt;

wobei ferner in Schritt (iii) so verfahren wird, dass

- die Temperatur der aus der zweiten Mischvorrichtung entnommenen Phosgenlösung auf einen Wert im Bereich von 0 °C bis 5 °C abgesenkt wird und
- der Druck im Entspannungsraum des Anlagenteils (IV) auf einen Wert im Bereich von 20 bar bis 25 bar ansteigt;

und wobei schließlich in Schritt (iv) so verfahren wird, dass

- die Temperatur der aus der ersten Mischvorrichtung entnommenen Aminlösung auf einen Wert im Bereich von 50 °C bis 60 °C abgesenkt wird,
- sich im Sollzustand eine Temperatur des aus der dritten Mischvorrichtung austretenden Reaktionsgemisches im Bereich von 128 °C bis 132 °C einstellt und
- der Druck im Entspannungsraum des Anlagenteils (IV) im Bereich von 20 bar bis 25 bar bleibt.

Vorzugsweise wird für den Druck im Entspannungsraum des Anlagenteils (IV), welcher *in Schritt (iii)* eingestellt wird, ein größerer Wert gewählt, als für den Druck in diesem Entspannungsraum, bei welchem *in Schritt (ii)* die kontinuierliche Zufuhr von Amin in die erste Mischvorrichtung gestartet wird. Hierdurch wird sichergestellt, dass die Reaktion auch beim Absenken des Phosgenüberschusses infolge der sukzessiven Erhöhung der Konzentration der zugeführten Anilinlösung keinesfalls zum Erliegen kommt.

Je nach den gewählten Bedingungen von Druck und Temperatur in Schritt (ii) wird gelöstes Phosgen teilweise aus der flüssigen Phase in den gasförmigen Zustand übergehen (ausgasen). Hierdurch steigt der Druck im Entspannungsraum des Anlagenteils (IV) an. Im erfindungsgemäßen Verfahren ist es vorgesehen, dass die Aminlösung und die Phosgenlösung erst dann erstmalig in der dritten Mischvorrichtung aufeinandertreffen, wenn der Druck im Entspannungsraum des Anlagenteils (IV) im Bereich von 16 bar bis 25 bar, bevorzugt 16 bar bis 20 bar, liegt (Startdruck). Hierzu ist die - bei gegebener Anlage leicht ermittelbare - Verweilzeit der Aminlösung vom Starten der Aminzufuhr in die erste Mischvorrichtung bis zum erstmaligen Eintreffen von Aminlösung in der dritten Mischvorrichtung zu berücksichtigen. Zusätzlich zu dem Druck, der sich ohne Ergreifen besonderer Maßnahmen durch die gegebenen Randbedingungen einstellt, kann der im Entspannungsraum des Anlagenteils (IV) noch durch Zugabe eines Inertgases (insbesondere Stickstoff) und/oder durch Zugabe von Chlorwasserstoff eingestellt werden. Chlorwasserstoff kann dabei in vorteilhafter Weise der ersten, zweiten und/oder dritten gasförmigen Phase entnommen werden, wobei solcher Chlorwasserstoff erforderlichenfalls auch aus einem früheren Produktionszeitraum zur Herstellung des Isocyanats (zwischengelagert in einem geeigneten Vorratsbehälter) stammen kann.

Nach Durchführung von Schritt (iv) befindet sich die Anlage im Regelbetrieb und kann bis zur nächsten Produktionsunterbrechung wie weiter oben beschrieben weiterbetrieben werden.

ABFAHREN DER PRODUKTION (ÜBERFÜHREN DER ANLAGE VOM SOLLZUSTAND IN EINEN ZUSTAND DES ANLAGENSTILLSTANDS)

Jede kontinuierliche Produktion muss von Zeit zu Zeit unterbrochen werden, sei es, weil die Nachfrage nach dem hergestellten Produkt gesunken ist oder Arbeiten (Wartung, Instandsetzung und dergleichen) an der Anlage notwendig sind. Zu diesem Zweck wird im Rahmen der vorliegenden Erfindung bevorzugt wie folgt vorgegangen:
Bei gegenüber dem Sollzustand (dem Regelbetrieb) unveränderten Mengenströmen an Phosgenlösung und Aminlösung werden zunächst die Temperaturen der Phosgen- und Aminlösungen *angehoben.* Hierdurch wird bewirkt, dass ein Temperaturabfall nach der dritten Mischvorrichtung, der eventuell eine Verunreinigung des Reaktors hervorrufen könnte, vermieden wird. Die Temperatur der Phosgenlösung wird von ihrem Soll-Wert für den Regelbetrieb im Bereich von 0 °C bis 10 °C auf bevorzugt einen Wert im Bereich von 100 °C bis 125 °C erhöht. Die Temperatur der Aminlösung wird von ihrem Soll-Wert für den Regelbetrieb im Bereich von 50 °C bis 80 °C bevorzugt auf einen Wert im Bereich von 100 °C bis 125 °C erhöht. Hierdurch steigt die Temperatur des aus der dritten Mischvorrichtung austretenden Reaktionsgemisches von ihrem Soll-Wert im Regelbetrieb im Bereich von 125 °C bis 135 °C auf höhere Werte an. Bei Erreichen einer Temperatur im Bereich von > 135 °C bis 140 °C, bevorzugt > 135 °C bis 137 °C, wird die weitere Zufuhr von Amin unterbrochen, während jedoch weiterhin ein Fluss an Lösungsmittel über die erste Mischvorrichtung in die dritte Mischvorrichtung aufrechterhalten wird, wobei dieser Fluss auch (zur Regulierung der Temperatur am Austritt der dritten Mischvorrichtung) höher oder geringer, insbesondere höher, sein kann als im Regelbetrieb. Mit zunehmender Verdünnung der Aminlösung fällt die Temperatur am Austritt der dritten Mischvorrichtung. Diese Temperatur wird bevorzugt im Bereich von 115 °C bis 125 °C gehalten.

Zum Spülen der eingesetzten Apparate und zum Abreagieren von in diesen noch vorhandenem Amin wird auch nach Abschaltung der Aminzufuhr weiterhin Phosgenlösung wie für einen gewissen Zeitraum über die zweite Mischvorrichtung der dritten Mischvorrichtung und den dieser nachgeschalteten Vorrichtungen zugeführt, bevorzugt für einen Zeitraum im Bereich von 15 Minuten bis 3 Stunden, zum Beispiel für einen Zeitraum von 20 Minuten. Nach Verstreichen dieses Zeitraums wird auch die Phosgenzufuhr unterbrochen und über die zweite Mischvorrichtung nur noch Lösungsmittel zum Verdünnen bzw. Auswaschen der noch in den Apparaten befindlichen Phosgenlösung in die dritte Mischvorrichtung und von dieser in die derselben nachgeschalteten Vorrichtungen gepumpt. Sobald das Phosgen hinreichend (d. h. bis zu einer zuvor festgelegten Grenzkonzentration) verdrängt ist, wird die Dosierung von Lösungsmittel über die zweite Mischvorrichtung unterbrochen. Danach (insbesondere 10 Minuten bis 20 Minuten, zum Beispiel 15 Minuten danach) wird auch die Zufuhr von Lösungsmittel über die erste Mischvorrichtung unterbrochen.

Wie weit Phosgen in der Anlage abgereichert werden muss, hängt von den Umständen ab. Bei einer vergleichsweise kurzen Produktionsunterbrechung ohne die Durchführung von Arbeiten an Phosgen-führenden Anlagenteilen ist eine vollständige Verdrängung des Phosgens im Allgemeinen nicht erforderlich. Soll die Anlage für längere Zeit außer Betrieb genommen werden oder sind Arbeiten an Phosgen-führenden Anlagenteilen erforderlich, so ist das Phosgen selbstverständlich vollständig zu verdrängen bzw. durch nachfolgende Reinigungsschritte wie beispielsweise eine Reaktion mit Ammoniak zu zersetzen.

Die Erfindung wird nachstehend anhand von Beispielen noch näher erläutert.

**Beispiele:**

Konzentrationsangaben in Prozent sind Massenprozent bezogen auf die Gesamtmasse des jeweiligen Stoffstroms. Für die Definition des Phosgenüberschusses siehe die Erläuterungen weiter oben.

Die Beispiele wurden in einer Miniplantanlage durchgeführt, in welcher im Sollzustand eine Aminlösung aus einer ersten Mischvorrichtung aus ca. 11,6 kg/h MDA und ca. 27 kg/h MCB mit einer Phosgenlösung aus einer zweiten Mischvorrichtung bei einem Phosgenüberschuss von ca. 60 % in einem dynamischen Mischer (dritte Mischvorrichtung) vermischt wurden und die resultierende Mischung einem adiabatisch betriebenen Blasensäulenreaktor (adiabatisch betriebener Reaktionsraum, erster Reaktor) zugeführt wurde. Das Produktgemisch aus dem Blasensäulenreaktor wurde in zwei Entspannungsvorrichtungen (erste Entspannungsvorrichtung entsprechend dem Entspannungsraum des Anlagenteils (IV) und zweite Entspannungsvorrichtung, Anlagenteil (V)) entspannt, und die erhaltene flüssige Phase wurde einem Fallfilmverdampfer (isotherm betriebener Reaktionsraum, zweiter Reaktor) zugeführt. Die in diesem anfallende flüssige Phase wurde einer Destillationskolonne zugeführt, in welcher MDI als Sumpfstrom und einer Phosgen- und Chlorwasserstoff-haltige Gasphase als Kopfstrom anfielen. Die in den beiden Entspannungsvorrichtungen anfallenden Gasphasen wurden mit dieser Gasphase vereinigt.

**Beispiel 1 (Vergleich - zu geringer Startdruck):**

Eine 60%ige Phosgenlösung wurde durch Vermischung von Monochlorbenzol (MCB) und Phosgen hergestellt und mit einem Massenstrom ṁ = 51,4 kg/h sowie einer Temperatur von 120 °C aus der zweiten Mischvorrichtung in dritte Mischvorrichtung gefördert. Anschließend wurde auf 95 °C erhitztes MCB mit einem Massenstrom ṁ = 30 kg/h über die erste Mischvorrichtung in die dritte Mischvorrichtung gefördert. Sodann begann man mit der Zufuhr von MDA in die zweite Mischvorrichtung und von dort in die dritte Mischvorrichtung. Es stellte sich eine Aminlösungstemperatur von 100 °C ein. Es wurde mit einer Aminlösungskonzentration von 25 % angefahren (entsprechend einer Menge von ca. 9,7 kg/h MDA und ca. 28,9 kg/h MCB). Nach ca. 7 Minuten erreichte die Aminlösung die dritte Mischvorrichtung, und die Reaktion startete. Der Druck im Entspannungsraum diesem Zeitpunkt (= Startdruck) betrug 15 bar. Durch die einsetzende Reaktion stieg die Temperatur am Austritt der dritten Mischvorrichtung innerhalb von 5 Minuten von 100 °C auf 135 °C. Sobald der Temperaturanstieg stoppte, wurde die Konzentration der Aminlösung auf 30 % erhöht (entsprechend einer Menge von ca. 11,6 kg/h MDA und ca. 27 kg/h MCB). Durch diese Änderung stieg die Temperatur am Austritt der dritten Mischvorrichtung auf 140 °C erreicht. Der Druck im Entspannungsraum stieg auf 20 bar an.

Anschließend wurde unter Berücksichtigung der Temperatur am Austritt der dritten Mischvorrichtung (diese sollte nicht unter 135 °C fallen) die Phosgenlösungstemperatur innerhalb von 90 Minuten von 120 °C auf 0 °C abgesenkt. Ca. 10 Minuten nach dem Start der Reduzierung der Phosgenlösungstemperatur wurde durch sukkzessive Erhöhung der Massenstroms und der Konzentration der Aminlösung (Endwert ca. 60 kg/h Aminlösung einer Konzentration von 30 %) der Phosgenüberschuss schrittweise von 210 % auf 60 % abgesenkt. Parallel dazu stieg der Druck im Entspannungsraum auf 26 bar. Das Absenken des Phosgenüberschusses (unter Beibehaltung einer Temperatur am Austritt der dritten Mischvorrichtung von mindestens 135 °C) dauerte ca. 60 Minuten.

Noch während des Absenkens des Phosgenüberschusses wurde eine geringere Gasentwicklung im Entspannungsraum beobachtet. Bei einem Druck von 24 bar im Entspannungsraum und einem Phosgenüberschuss von ca. 60 % erfolgte ein Temperaturabfall am Austritt der dritten Mischvorrichtung (was ein "Einschlafen" der Reaktion anzeigt). Der Versuch musste beendet werden, um ein Verschmutzen des Reaktors zu vermeiden.

**Beispiel 2 (Vergleich - Startdruck ausreichend hoch, jedoch Temperatur am Austritt der dritten Mischvorrichtung zu hoch):**

Eine 60%ige Phosgenlösung wurde durch Vermischung von Monochlorbenzol (MCB) und Phosgen hergestellt und mit einem Massenstrom ṁ = 51,4 kg/h sowie einer Temperatur von 120 °C aus der zweiten Mischvorrichtung in dritte Mischvorrichtung gefördert. Anschließend wurde auf 80 °C erhitztes MCB mit einem Massenstrom ṁ = 30 kg/h über die erste Mischvorrichtung in die dritte Mischvorrichtung gefördert. Sodann begann man mit der Zufuhr von MDA in die zweite Mischvorrichtung und von dort in die dritte Mischvorrichtung. Es stellte sich eine Aminlösungstemperatur von 95 °C ein. Es wurde mit einer Aminlösungskonzentration von 25 % angefahren (entsprechend einer Menge von ca. 9,7 kg/h MDA und ca. 28,9 kg/h MCB). Nach ca. 7 Minuten erreichte die Aminlösung die dritte Mischvorrichtung, und die Reaktion startete. Der Druck im Entspannungsraum betrug vor dem Starten der Reaktion 15 bar und wurde rechtzeitig vor dem erstmaligen Aufeinandertreffen von MDA und Phosgen durch Stickstoffzugabe auf 20 bar erhöht (= Startdruck). Durch die einsetzende Reaktion stieg die Temperatur am Austritt der dritten Mischvorrichtung innerhalb von 5 Minuten von 100 °C auf 135 °C. Sobald der Temperaturanstieg stoppte, wurde die Konzentration der Aminlösung auf 30 % erhöht (entsprechend einer Menge von ca. 11,6 kg/h MDA und ca. 27 kg/h MCB). Durch diese Änderung stieg die Temperatur am Austritt der dritten Mischvorrichtung auf 140 °C erreicht. Der Druck im Entspannungsraum stieg als Folge des Reaktionsfortschritts innerhalb von 10 Minuten auf 26 bar.

Anschließend wurde ohne Berücksichtigung der Temperatur am Austritt der dritten Mischvorrichtung die Phosgenlösungstemperatur innerhalb von 90 Minuten von 120 °C auf 0 °C abgesenkt. Ca. 10 Minuten nach dem Start der Reduzierung der Phosgenlösungstemperatur wurde durch sukzessive Erhöhung der Massenstroms und der Konzentration der Aminlösung (Endwert ca. 60 kg/h Aminlösung einer Konzentration von 30 %) der Phosgenüberschuss schrittweise von 210 % auf 60 % abgesenkt. Das Absenken des Phosgenüberschusses (wiederum ohne Berücksichtigung der Temperatur am Austritt der dritten Mischvorrichtung) dauerte ca. 30 Minuten. Es kam zu einem Temperaturanstieg am Austritt der dritten Mischvorrichtung auf 150 °C, was mit vermehrter Feststoffbildung und einem Belegen der Reaktorinnenwände mit Feststoff einherging. Der Versuch wurde beendet, um ein weiteres Verschmutzen des Reaktors zu vermeiden.

**Beispiel 3 (erfindungsgemäß - Startdruck ausreichend hoch und Temperatur am Austritt der dritten Mischvorrichtung im geeigneten Bereich):**

Eine 60%ige Phosgenlösung wurde durch Vermischung von Monochlorbenzol (MCB) und Phosgen hergestellt und mit einem Massenstrom ṁ = 51,4 kg/h sowie einer Temperatur von 120 °C aus der zweiten Mischvorrichtung in dritte Mischvorrichtung gefördert. Anschließend wurde auf 80 °C erhitztes MCB mit einem Massenstrom ṁ = 30 kg/h über die erste Mischvorrichtung in die dritte Mischvorrichtung gefördert. Sodann begann man mit der Zufuhr von MDA in die zweite Mischvorrichtung und von dort in die dritte Mischvorrichtung. Es stellte sich eine Aminlösungstemperatur von 95 °C ein. Es wurde mit einer Aminlösungskonzentration von 25 % angefahren (entsprechend einer Menge von ca. 9,7 kg/h MDA und ca. 28,9 kg/h MCB). Nach ca. 7 Minuten erreichte die Aminlösung die dritte Mischvorrichtung, und die Reaktion startete. Der Druck im Entspannungsraum betrug vor dem Starten der Reaktion 15 bar und wurde rechtzeitig vor dem erstmaligen Aufeinandertreffen von MDA und Phosgen durch Stickstoffzugabe auf 20 bar erhöht (= Startdruck). Durch die einsetzende Reaktion stieg die Temperatur am Austritt der dritten Mischvorrichtung innerhalb von 5 Minuten von 100 °C auf 135 °C. Sobald der Temperaturanstieg stoppte, wurde die Konzentration der Aminlösung auf 30 % erhöht (entsprechend einer Menge von ca. 11,6 kg/h MDA und ca. 27 kg/h MCB). Durch diese Änderung stieg die Temperatur am Austritt der dritten Mischvorrichtung auf 140 °C erreicht. Der Druck im Entspannungsraum stieg als Folge des Reaktionsfortschritts innerhalb von 10 Minuten auf 26 bar.

Anschließend wurde unter Berücksichtigung der Temperatur am Austritt der dritten Mischvorrichtung (diese sollte bei ca. 140 °C bleiben) die Phosgenlösungstemperatur innerhalb von 90 Minuten von 120 °C auf 0 °C abgesenkt. Ca. 10 Minuten nach dem Start der Reduzierung der Phosgenlösungstemperatur wurde durch sukzessive Erhöhung der Massenstroms und der Konzentration der Aminlösung (Endwert ca. 60 kg/h Aminlösung einer Konzentration von 30 %) der Phosgenüberschuss schrittweise von 210 % auf 60 % abgesenkt. Das Absenken des Phosgenüberschusses (unter Beibehaltung einer Temperatur am Austritt der dritten Mischvorrichtung von etwa 140 °C) dauerte ca. 30 Minuten.

Nach Erreichen eines Phosgenüberschusses von 60 % wurde die Aminlösungstemperatur innerhalb von 30 Minuten von 95 °C auf 55 °C reduziert. Unter diesen Bedingungen (Aminlösungstemperatur 55 °C, Phosgenlösungstemperatur 0 °C, Phosgenüberschuss 60 %, Druck im Entspannungsraum 26 bar) stellt sich eine Temperatur des Reaktionsgemisches am Austritt der dritten Mischvorrichtung von 130 °C ein. Die Anlage befindet sich damit im Sollzustand. Der Zeitraum von Beginn des Anfahrens bis zum Erreichen des Sollzustandes betrug ca. 4 Stunden.

**Patentansprüche**

1.    Verfahren zum Betreiben einer Anlage zur kontinuierlichen Herstellung eines Isocyanats durch Umsetzung eines primären Amins A mit Phosgen P unter Einhaltung eines auf die Aminogruppen des primären Amins bezogenen stöchiometrischen Überschusses an Phosgen in Gegenwart eines Lösungsmittels L in der Flüssigphase,

  wobei die Anlage die folgenden Anlagenteile aufweist:

    (I) eine erste Mischvorrichtung ausgelegt zur Vermischung von primärem Amin A und Lösungsmittel L zu einer Aminlösung AL,
    (II) eine zweite Mischvorrichtung ausgelegt zur Vermischung von Phosgen P und Lösungsmittel L zu einer Phosgenlösung PL,
    (III) eine dritte Mischvorrichtung ausgelegt zur Vermischung der Aminlösung und der Phosgenlösung zu einem Reaktionsgemisch,
    (IV) einen ersten Reaktionsraum ausgelegt zur adiabatischen Umsetzung des Reaktionsgemisches und einen dem Reaktionsraum nachgeschalteten Entspannungsraum ausgelegt zur Ausbildung einer ersten flüssigen Phase und einer ersten gasförmigen Phase,
    (V) eine Entspannungsvorrichtung zum Entspannen der ersten flüssigen Phase unter Ausbildung einer zweiten flüssigen Phase und einer zweiten gasförmigen Phase,
    (VI) einen zweiten Reaktionsraum ausgelegt zur isothermen Umsetzung der zweiten flüssigen Phase unter Ausbildung einer dritten flüssigen Phase und einer dritten gasförmigen Phase,
    (VII) eine Aufarbeitungseinheit zur Gewinnung des Isocyanats aus der dritten flüssigen Phase;

  wobei das Verfahren in einem Sollzustand die kontinuierliche Umsetzung

    der Aminlösung in einer Soll-Konzentration an Amin in der Aminlösung von $c(A)_{SOLL}$ und einem Soll-Mengenstrom der Aminlö-

sung von $\dot{m}(AL)_{SOLL}$, welcher sich aus einem Soll-Mengenstrom an Amin von $\dot{m}(A)_{SOLL}$ und einem ersten Soll-Mengenstrom an Lösungsmittel von $\dot{m}(LA)_{SOLL}$ ergibt,

mit der Phosgenlösung in einer Soll-Konzentration an Phosgen in der Phosgenlösung von $c(P)_{SOLL}$ und einem Soll-Mengenstrom der Phosgenlösung von $\dot{m}(PL)_{SOLL}$, welcher sich aus einem Soll-Mengenstrom an Phosgen von $\dot{m}(P)_{SOLL}$ und einem zweiten Soll-Mengenstrom an Lösungsmittel von $\dot{m}(LP)_{SOLL}$ ergibt,

umfasst, und wobei

ausgehend von einem Zustand, in dem der dritten Mischvorrichtung keine Aminlösung zugeführt wird, folgende Schritte zur Erreichung des Sollzustandes durchgeführt werden:

(i) kontinuierliches Zuführen

(a) von Phosgenlösung einer Temperatur im Bereich von 100 °C bis 125 °C in der Soll-Konzentration $c(P)_{SOLL}$ und dem Soll-Mengenstrom $\dot{m}(PL)_{SOLL}$ aus der zweiten Mischvorrichtung und
(b) von Lösungsmittel einer Temperatur im Bereich von 70 °C bis 100 °C in dem ersten Soll-Mengenstrom $\dot{m}(LA)_{SOLL}$ aus der ersten Mischvorrichtung

in die dritte Mischvorrichtung und von dort durch den ersten Reaktionsraum, den Entspannungsraum, die Entspannungsvorrichtung und den zweiten Reaktionsraum in die Aufarbeitungseinheit,

(ii) Starten der kontinuierlichen Zufuhr von Amin in die erste Mischvorrichtung, durch welche weiterhin der in Schritt (i)(b) etablierte Strom an Lösungsmittel fließt und sich so eine Aminlösung ausbildet, mit einem Start-Mengenstrom an Amin von $\dot{m}(A)_{START}$, welcher kleiner ist als der Soll-Mengenstrom an Amin $\dot{m}(A)_{SOLL}$, sodass sich eine Temperatur der die erste Mischvorrichtung verlassenden Aminlösung im Bereich von 85 °C bis 105 °C einstellt und sich eine Temperatur des aus der dritten Mischvorrichtung austretenden Reaktionsgemisches im Bereich von 130 °C bis 145 °C ergibt, wobei der Zeitpunkt des Startens der kontinuierlichen Zufuhr von Amin in die erste Mischvorrichtung so gewählt wird, dass beim erstmaligem Aufeinandertreffen von Aminlösung und Phosgenlösung in der dritten Mischvorrichtung ein Druck im Entspannungsraum des Anlagenteils (IV) im Bereich von 16 bar bis 25 bar vorliegt,

(iii) Erhöhen des Mengenstroms an Amin von $\dot{m}(A)_{START}$ auf $\dot{m}(A)_{SOLL}$ (in Stufen oder kontinuierlich) und parallel dazu Absenken der Temperatur der aus der zweiten Mischvorrichtung entnommenen Phosgenlösung auf einen Wert im Bereich von 0 °C bis 10 °C derart, dass die Temperatur des aus der dritten Mischvorrichtung austretenden Reaktionsgemisches im Bereich von 130 °C bis 145 °C bleibt und der Druck im Entspannungsraum des Anlagenteils (IV) im Bereich von 20 bar bis 30 bar liegt,

(iv) nach Erreichen des Soll-Mengenstroms an Amin (d. h. $\dot{m}(A) = \dot{m}(A)_{Soll}$): Absenken der Temperatur der aus der ersten Mischvorrichtung entnommenen Aminlösung auf einen Wert im Bereich von 50 °C bis 80 °C sodass sich im Sollzustand eine Temperatur des aus der dritten Mischvorrichtung austretenden Reaktionsgemisches im Bereich von 125 °C bis 135 °C einstellt und der Druck im Entspannungsraum des Anlagenteils (IV) im Bereich von 20 bar bis 30 bar bleibt.

2. Verfahren nach Anspruch 1,

bei welchem in Schritt (i)

• die Temperatur der Phosgenlösung auf einen Wert im Bereich von 100 °C bis 105 °C und
• die Temperatur des Lösungsmittels auf einen Wert im Bereich von 90 °C bis 95 °C, eingestellt werden;

und bei welchem in Schritt (ii)

• der Druck im Entspannungsraum des Anlagenteils (IV) beim erstmaligem Aufeinandertreffen von Aminlösung und Phosgenlösung in der dritten Mischvorrichtung im Bereich von 16 bar bis 20 bar liegt;
• sich eine Temperatur der die erste Mischvorrichtung verlassenden Aminlösung im Bereich von 95 °C bis 100 °C, einstellt und
• sich eine Temperatur des aus der dritten Mischvorrichtung austretenden Reaktionsgemisches im Bereich von 138 °C bis 142 °C ergibt;

und bei welchem in Schritt (iii)

• die Temperatur der aus der zweiten Mischvorrichtung entnommenen Phosgenlösung auf einen Wert im Bereich von 0 °C bis 5 °C abgesenkt wird und

• der Druck im Entspannungsraum des Anlagenteils (IV) auf einen Wert im Bereich von 20 bar bis 25 bar ansteigt;

und bei welchem in Schritt (iv)

• die Temperatur der aus der ersten Mischvorrichtung entnommenen Aminlösung auf einen Wert im Bereich von 50 °C bis 60 °C abgesenkt wird,
• sich im Sollzustand eine Temperatur des aus der dritten Mischvorrichtung austretenden Reaktionsgemisches im Bereich von 128 °C bis 132 °C einstellt und
• der Druck im Entspannungsraum des Anlagenteils (IV) im Bereich von 20 bar bis 25 bar bleibt.

3. Verfahren nach Anspruch 1 oder 2, bei welchem der Druck im Entspannungsraum des Anlagenteils (IV), welcher in Schritt (iii) eingestellt wird, größer ist als der Druck im Entspannungsraum des Anlagenteils (IV), bei welchem in Schritt (ii) die kontinuierliche Zufuhr von Amin in die erste Mischvorrichtung gestartet wird.

4. Verfahren nach einem der Ansprüche 1 bis 3, bei welchem der Reaktionsraum und der Entspannungsraum des Anlagenteils (IV) in zwei verschiedenen Vorrichtungen angeordnet sind.

5. Verfahren nach einem der Ansprüche 1 bis 3, bei welchem der Reaktionsraum und der Entspannungsraum des Anlagenteils (IV) in einer gemeinsamen Vorrichtung angeordnet sind.

6. Verfahren nach einem der vorstehenden Ansprüche, bei welchem in Schritt (ii) der Druck im Entspannungsraum des Anlagenteils (IV) durch ausgasendes Phosgen, durch Zugabe eines Inertgases und/oder durch Zugabe von Chlorwasserstoff eingestellt wird.

7. Verfahren nach Anspruch 6, umfassend die Einstellung des Drucks im Entspannungsraum des Anlagenteils (IV) durch Zugabe von Chlorwasserstoff, wobei der Chlorwasserstoff der ersten, zweiten und/oder dritten gasförmigen Phase entnommen wird.

8. Verfahren nach einem Ansprüche 1 bis 7, bei welchem mit der Durchführung von Schritt (i)(b) gleichzeitig mit Schritt (i)(a) begonnen wird.

9. Verfahren nach einem Ansprüche 1 bis 7, bei welchem mit der Durchführung von Schritt (i)(b) vor Schritt (i)(a) begonnen wird.

10. Verfahren nach einem Ansprüche 1 bis 7, bei welchem mit der Durchführung von Schritt (i)(b) nach Schritt (i)(a) begonnen wird.

11. Verfahren nach einem der vorstehenden Ansprüche, bei welchem die Aufarbeitungseinheit

• eine erste Destillationsvorrichtung ausgelegt zur Abtrennung einer Phosgen-haltigen vierten gasförmigen Phase aus der dritten flüssigen Phase unter Erhalt einer an Phosgen abgereicherten vierten flüssigen Phase,
• eine zweite Destillationsvorrichtung ausgelegt zur Abtrennung einer Lösungsmittelhaltigen fünften gasförmigen Phase aus der vierten flüssigen Phase unter Erhalt einer an Phosgen und Lösungsmittel abgereicherten fünften flüssigen Phase und
• eine dritte Destillationsvorrichtung ausgelegt zur Gewinnung des Isocyanats aus der fünften flüssigen Phase umfasst.

12. Verfahren nach einem der vorstehenden Ansprüche, bei welchem nach Erreichen des Sollzustandes die kontinuierliche Umsetzung der Aminlösung mit der Phosgenlösung unterbrochen wird, wobei zur Unterbrechung die folgenden Schritte durchlaufen werden:

(v) Anheben der Temperatur der Phosgenlösung und der Temperatur der Aminösung bei gegenüber dem Sollzustand unveränderten Mengenströmen an Phosgenlösung und Aminlösung;
(vi) bei Erreichen einer Temperatur des aus der dritten Mischvorrichtung austretenden Reaktionsgemisches im Bereich von > 135 °C bis 140 °C:
Abstellen der Zufuhr von Amin in die erste Mischvorrichtung unter Beibehaltung der Zufuhr von Lösungsmittel in die erste Mischvorrichtung und unter Beibehaltung der Zufuhr von Phosgenlösung in die zweite Mischvorrichtung;
(vii) Abstellen der Zufuhr von Phosgen in die zweite Mischvorrichtung unter Beibehaltung der Zufuhr von Lösungsmittel in die zweite Mischvorrichtung und unter Beibehaltung der Zufuhr von Lösungsmittel in die erste Mischvorrichtung;
(viii) nach Unterschreiten einer zuvor festgelegten Grenzkonzentration an Phosgen in dem Lösungsmittel, das aus der zweiten Mischvorrichtung austritt:
Abstellen der Zufuhr von Lösungsmittel in die zweite Mischvorrichtung; und
(viii) Abstellen der Zufuhr von Lösungsmittel in die erste Mischvorrichtung.

13. Verfahren nach Anspruch 12, bei welchem in Schritt (v) die Temperatur der Phosgenlösung auf einen Wert im Bereich von 100 °C bis 125 °C erhöht wird, und bei welchem die Temperatur der Aminlösung auf einen Wert im Bereich von 100 °C bis 125 °C erhöht wird.

14. Verfahren nach Anspruch 12 oder 13, bei welchem in Schritt (vi) die Aminzufuhr abgeschaltet wird, sobald die Temperatur des aus der dritten Mischvorrichtung austretenden Reaktionsgemisches im Bereich von > 135 °C bis 137 °C liegt.

15. Verfahren nach einem der vorstehenden Ansprüche, bei welchem das primäre Amin ausgewählt ist aus Methylen-Diphenylen-Diamin, Polymethylen-Polyphenylen-Polyamin, einem Gemisch aus Methylen-Diphenylen-Diamin und Polymethylen-Polyphenylen-Polyamin, Toluylendiamin, Xylylendiamin, 1,5-Pentandiamin, 1,6-Hexamethylendiamin, Isophorondiamin und Naphthyldiamin.

**Claims**

1. Process for operating a plant for continuous preparation of an isocyanate by reaction of a primary amine A with phosgene P while maintaining a stoichiometric excess of phosgene based on the amino groups of the primary amine in the presence of a solvent S in the liquid phase,

   wherein the plant comprises the following plant parts:

      (I) a first mixing apparatus configured for mixing primary amine A and solvent S to afford an amine solution AS,
      (I) a second mixing apparatus configured for mixing phosgene P and solvent S to afford a phosgene solution PS,
      (III) a third mixing apparatus configured for mixing the amine solution and the phosgene solution to afford a reaction mixture,
      (IV) a first reaction space configured for adiabatic reaction of the reaction mixture and a decompression space arranged downstream of the reaction space configured to form a first liquid phase and a first gaseous phase,
      (V) a decompression apparatus for decompressing the first liquid phase to form a second liquid phase and a second gaseous phase,
      (VI) a second reaction space configured for isothermal reaction of the second liquid phase to form a third liquid phase and a third gaseous phase,
      (VII) a workup unit to obtain the isocyanate from the third liquid phase;

wherein in a target state the process comprises the continuous reaction

   of the amine solution in a target concentration of amine in the amine solution of $c(A)_{TARGET}$ and a target flow rate of the amine solution of $\dot{m}(AS)_{TARGET}$ which results from a target flow rate of amine of $\dot{m}(A)_{TARGET}$ and a first target flow rate of solvent of $m(SA)_{TARGET}$,
   with the phosgene solution in a target concentration of phosgene in the phosgene solution of $c(P)_{TARGET}$ and a target flow rate of the phosgene solution of $\dot{m}(PS)_{TARGET}$ which results from a target flow rate of phosgene of $\dot{m}(P)_{TARGET}$ and a second target flow rate of solvent of $\dot{m}(SP)_{TARGET}$,

and wherein
starting from a state in which no amine solution is supplied to the third mixing apparatus the following steps are performed to achieve the target state:

   (i) continuously supplying

      (a) phosgene solution having a temperature in the range from 100°C to 125° at the target concentration $c(P)_{TARGET}$ and the target flow rate $\dot{m}(PS)_{TARGET}$ from the second mixing apparatus and (b) solvent having a temperature in the range from 70°C to 100°C at the first target flow rate $\dot{m}(SA)_{TARGET}$ from the first mixing apparatus

   into the third mixing apparatus and from there through the first reaction space, the decompression space, the decompression apparatus and the second reaction space into the workup unit,
   (ii) starting the continuous supply of amine into the first mixing apparatus, through which the stream of solvent established in step (i)(b) continues to flow, thus forming an amine solution, at a starting flow rate of amine of $\dot{m}(A)_{START}$ which is less than the target flow rate of amine $\dot{m}(A)_{TARGET}$, so that a temperature of the amine solution exiting the first mixing apparatus in the range from 85°C to 105°C results and a temperature of the reaction mixture exiting the third mixing apparatus in the range from 130°C to 145°C is obtained, wherein the point in time of starting the continuous supply of amine into the first mixing apparatus is chosen such that upon first contacting of the amine solution and the phosgene solution in the third mixing apparatus the pressure in the decompression space of plant part (IV) is in the range from 16 to 25 bar,

(iii) increasing the flow rate of amine from $\dot{m}(A)_{START}$ to $\dot{m}(A)_{TARGET}$ (in steps or continuously) and simultaneously reducing the temperature of the phosgene solution withdrawn from the second mixing apparatus to a value in the range from 0°C to 10°C in such a way that the temperature of the reaction mixture exiting the third mixing apparatus remains in the range from 130°C to 145°C and the pressure in the decompression space of plant part (IV) is in the range from 20 bar to 30 bar,

(iv) after achieving the target flow rate of amine (i.e. $\dot{m}(A) = \dot{m}(A)_{TARGET}$): reducing the temperature of the amine solution withdrawn from the first mixing apparatus to a value in the range from 50°C to 80°C so that in the target state a temperature of the reaction mixture exiting the third mixing apparatus in the range from 125°C to 135°C results and the pressure in the decompression space of plant part (IV) remains in the range from 20 bar to 30 bar.

2. Process according to Claim 1,

wherein in step (i)

• the temperature of the phosgene solution is set to a value in the range from 100°C to 105°C and
• the temperature of the solvent is set to a value in the range from 90°C to 95°C;

and wherein in step (ii)

• the pressure in the decompression space of plant part (IV) upon first contacting of the amine solution and the phosgene solution in the third mixing apparatus is in the range from 16 bar to 20 bar;
• a temperature of the amine solution exiting the first mixing apparatus in the range from 95°C to 100°C results and
• a temperature of the reaction mixture exiting the third mixing apparatus in the range from 138°C to 142°C is obtained;

and wherein in step (iii)

• the temperature of the phosgene solution withdrawn from the second mixing apparatus is reduced to a value in the range from 0°C to 5°C and
• the pressure in the decompression space of plant part (IV) increases to a value in the range from 20 bar to 25 bar;

and wherein in step (iv)

• the temperature of the amine solution withdrawn from the first mixing apparatus is reduced to a value in the range from 50°C to 60°C,
• in the target state a temperature of the reaction mixture exiting the third mixing apparatus in the range from 128°C to 132°C results and
• the pressure in the decompression space of plant part (IV) remains in the range from 20 bar to 25 bar.

3. Process according to Claim 1 or 2, wherein the pressure in the decompression space of plant part (IV), which is established in step (iii), is greater than the pressure in the decompression space of plant part (IV) at which the continuous supplying of amine into the first mixing apparatus is started in step (ii).

4. Process according to any of Claims 1 to 3, wherein the reaction space and the decompression space of plant part (IV) are arranged in two different apparatuses.

5. Process according to any of Claims 1 to 3, wherein the reaction space and the decompression space of plant part (IV) are arranged in a common apparatus.

6. Process according to any of the preceding claims, wherein in step (ii) the pressure in the decompression space of plant part (IV) is adjusted by outgassing phosgene, by addition of an inert gas and/or by addition of hydrogen chloride.

7. Process according to Claim 6, comprising adjusting the pressure in the decompression space of plant part (IV) by addition of hydrogen chloride, wherein the hydrogen chloride is withdrawn from the first, second and/or third gaseous phase.

8. Process according to any of Claims 1 to 7, wherein the performance of step (i) (b) is commenced simultaneously with step (i)(a).

9. Process according to any of Claims 1 to 7, wherein the performance of step (i)(b) is commenced before step (i) (a) .

10. Process according to any of Claims 1 to 7, wherein the performance of step (i) (b) is commenced after step (i) (a) .

**11.** Process according to any of the preceding claims, wherein the workup unit comprises

• a first distillation apparatus configured for separating a phosgene-containing fourth gaseous phase from the third liquid phase to obtain a fourth liquid phase depleted in phosgene,
• a second distillation apparatus configured for separating a solvent-containing fifth gaseous phase from the fourth liquid phase to obtain a fifth liquid phase depleted in phosgene and solvent and
• a third distillation apparatus configured for obtaining the isocyanate from the fifth liquid phase.

**12.** Process according to any of the preceding claims, wherein after achieving the target state the continuous reaction of the amine solution with the phosgene solution is interrupted, wherein the interruption is effected by performing the following steps:

(v) increasing the temperature of the phosgene solution and the temperature of the amine solution at flow rates of phosgene solution and amine solution unchanged relative to the target state;
(vi) upon achieving a temperature of the reaction mixture exiting the third mixing apparatus in the range from > 135°C to 140°C:
terminating the supply of amine into the first mixing apparatus while maintaining the supply of solvent into the first mixing apparatus and maintaining the supply of phosgene solution into the second mixing apparatus;
(vii) terminating the supply of phosgene into the second mixing apparatus while maintaining the supply of solvent into the second mixing apparatus and while maintaining the supply of solvent into the first mixing apparatus;
(viii) after falling below a previously specified threshold concentration of phosgene in the solvent exiting the second mixing apparatus:
terminating the supply of solvent into the second mixing apparatus; and
(viii) terminating the supply of solvent into the first mixing apparatus.

**13.** Process according to Claim 12, wherein in step (v) the temperature of the phosgene solution is increased to a value in the range from 100°C to 125°C and wherein the temperature of the amine solution is increased to a value in the range from 100°C to 125°C.

**14.** Process according to Claim 12 or 13, wherein in step (vi) the amine supply is terminated as soon as the temperature of the reaction mixture exiting the third mixing apparatus is in the range from > 135°C to 137°C.

**15.** Process according to any of the preceding claims, wherein the primary amine is selected from methylene diphenylene diamine, polymethylene polyphenylene polyamine, a mixture of methylene diphenylene diamine and polymethylene polyphenylene polyamine, tolylene diamine, xylylene diamine, pentane-1,5-diamine, hexamethylene-1,6-diamine, isophoronediamine and naphthyldiamine.

**Revendications**

**1.** Procédé de fonctionnement d'une installation destinée à la fabrication continue d'un isocyanate par réaction d'une amine primaire A avec du phosgène P tout en maintenant un excédent stoechiométrique de phosgène par rapport aux groupes amino de l'amine primaire en présence d'un solvant S dans la phase liquide,

dans lequel l'installation comporte les parties d'installation suivantes :

(I) un premier dispositif de mélange conçu pour mélanger l'amine primaire A et le solvant S pour former une solution d'amine AS,
(II) un deuxième dispositif de mélange conçu pour mélanger le phosgène P et le solvant S pour former une solution de phosgène PS,
(III) un troisième dispositif de mélange conçu pour mélanger la solution d'amine et la solution de phosgène pour former un mélange réactionnel,
(IV) un premier espace de réaction conçu pour la réaction adiabatique du mélange réactionnel et un espace de détente raccordé en aval de l'espace de réaction conçu pour former une première phase liquide et une première phase gazeuse,
(V) un dispositif de détente pour détendre la première phase liquide pour former une deuxième phase liquide et une deuxième phase gazeuse,
(VI) un second espace de réaction conçu pour la réaction isotherme de la deuxième phase liquide pour former une troisième phase liquide et une troisième phase gazeuse,
(VII) une unité de traitement pour recueillir l'isocyanate de la troisième phase liquide ;

le procédé comprenant, dans un état cible, la réaction continue de la solution d'amine en une concentration cible d'amine dans la solution d'amine de $c(A)_{CIBLE}$ et un débit cible de la solution d'amine de $\dot{m}(AS)_{CIBLE}$, qui consiste en un débit cible d'amine de $\dot{m}(A)_{CIBLE}$ et en un premier débit cible de solvant de $\dot{m}(SA)_{CIBLE}$, avec la solution de phosgène en une concentration cible de phosgène dans la solution de phosgène de $c(P)_{CIBLE}$ et un débit cible de la solution de phosgène de $\dot{m}(PS)_{CIBLE}$, qui consiste en un débit cible de phosgène de $\dot{m}(P)_{CIBLE}$ et en un second débit cible de solvant de $\dot{m}(SP)_{CIBLE}$,

et à partir d'un état dans lequel aucune solution d'amine n'est fournie au troisième dispositif de mélange, les étapes suivantes étant réalisées pour obtenir l'état cible :

(i) l'alimentation continue

(a) en solution de phosgène d'une température se situant dans la plage de 100 °C à 125 °C dans la concentration cible $c(P)_{CIBLE}$ et le débit cible $\dot{m}(PS)_{CIBLE}$ provenant du deuxième dispositif de mélange et

(b) en solvant d'une température se situant dans la plage de 70 °C à 100 °C dans le premier débit cible $\dot{m}(SA)_{CIBLE}$ provenant du premier dispositif de mélange

dans le troisième dispositif de mélange et de là, en passant par le premier espace de réaction, l'espace de détente, le dispositif de détente et le second espace de réaction, jusque dans l'unité de traitement,

(ii) le démarrage de l'alimentation continue en amine dans le premier dispositif de mélange à travers lequel le flux de solvant établi à l'étape (i)(b) continue de s'écouler et forme ainsi une solution d'amine, avec un débit initial d'amine de $\dot{m}(A)_{INITIAL}$ qui est plus petit que le débit cible d'amine $\dot{m}(A)_{CIBLE}$, de façon qu'une température de la solution d'amine sortant du premier dispositif de mélange dans la plage de 85 °C à 105 °C en résulte et qu'une température du mélange réactionnel sortant du troisième dispositif de mélange se situe dans la plage de 130 °C à 145 °C est obtenue, le moment du démarrage de l'alimentation continue en amine dans le premier dispositif de mélange étant choisi de façon que, lorsque la solution d'amine et la solution de phosgène entrent en contact pour la première fois dans le troisième dispositif de mélange, une pression dans l'espace de détente de la partie d'installation (IV) se situe dans la plage de 16 bar à 25 bar,

(iii) l'augmentation du débit d'amine de $\dot{m}(A)_{INITIAL}$ à $\dot{m}(A)_{CIBLE}$ (par étapes ou en continu) et en parallèle, la réduction de la température de la solution de phosgène retirée du deuxième dispositif de mélange jusqu'à une valeur située dans la plage de 0 °C à 10 °C, de façon que la température du mélange réactionnel sortant du troisième dispositif de mélange demeure dans la plage de 130 °C et 145 °C et la pression dans l'espace de détente de la partie d'installation (IV) se situe dans la plage de 20 bar à 30 bar,

(iv) après avoir atteint le débit cible d'amine (à savoir $\dot{m}(A) = \dot{m}(A)_{CIBLE}$), l'abaissement de la température de la solution d'amine retirée du premier dispositif de mélange jusqu'à une valeur de 50 °C à 80 °C, de façon que, dans l'état cible, une température du mélange réactionnel sortant du troisième dispositif de mélange dans la plage de 125 °C à 135 °C en résulte et la pression dans l'espace de détente de la partie d'installation (IV) demeure dans la plage de 20 bar à 30 bar.

2. Procédé selon la revendication 1,

dans lequel, à l'étape (i)

• la température de la solution de phosgène est réglée à une valeur située dans la plage de 100 °C à 105 °C et
• la température du solvant est réglée à une valeur située dans la plage de 90 °C à 95 °C ;

et dans lequel, à l'étape (ii)

• la pression dans l'espace de détente de la partie d'installation (IV), lorsque la solution d'amine et la solution de phosgène entrent en contact pour la première fois dans le troisième dispositif de mélange, se situe dans la plage de 16 bar à 20 bar ;
• une température de la solution d'amine sortant du premier dispositif de mélange dans la plage de 95 °C à 100 °C en résulte et
• une température du mélange réactionnel sortant du troisième dispositif de mélange dans la plage de 138 °C à 142 °C est obtenue ;

et dans lequel à l'étape (iii)

• la température de la solution de phosgène retirée du deuxième mélange est abaissée à une valeur située dans la plage de 0 °C à 5 °C et
• la pression dans l'espace de détente de la partie d'installation (IV) augmente à une valeur située dans la plage de 20 bar à 25 bar ;

et dans lequel à l'étape (iv)

• la température de la solution d'amine retirée du premier dispositif de mélange est abaissée à une valeur située dans la plage de 50 °C à 60 °C,
• dans l'état cible, une température du mélange réactionnel sortant du troisième dispositif de mélange dans la plage de 128 °C à 132 °C en résulte et
• la pression dans l'espace de détente de la partie d'installation (IV) demeure dans la plage de 20 bar à 25 bar.

3. Procédé selon la revendication 1 ou 2, dans lequel la pression dans l'espace de détente de la partie d'installation (IV) réglée à l'étape (iii) est supérieure à la pression dans l'espace de détente de la partie d'installation (IV), dans lequel à l'étape (ii), l'alimentation continue d'amine démarre dans le premier dispositif de mélange.

4. Procédé selon l'une quelconque des revendications 1 à 3, dans lequel l'espace de réaction et l'espace de détente de la partie d'installation (IV) sont agencés dans deux dispositifs différents.

5. Procédé selon l'une quelconque des revendications 1 à 3, dans lequel l'espace de réaction et l'espace de détente de la partie d'installation (IV) sont agencés dans un dispositif commun.

6. Procédé selon l'une quelconque des revendications précédentes, dans lequel, à l'étape (ii), la pression dans l'espace de détente de la partie d'installation (IV) est réglée par dégazage du phosgène, par ajout d'un gaz inerte et/ou par ajout de chlorure d'hydrogène.

7. Procédé selon la revendication 6, comprenant le réglage de la pression dans l'espace de détente de la partie d'installation (IV) par ajout de chlorure d'hydrogène, le chlorure d'hydrogène étant retiré de la première, de la deuxième et/ou de la troisième phase gazeuse.

8. Procédé selon l'une quelconque des revendications 1 à 7, dans lequel la mise en œuvre de l'étape (i) (b) démarre en même temps que l'étape (i)(a).

9. Procédé selon l'une quelconque des revendications 1 à 7, dans lequel la mise en œuvre de l'étape (i) (b) démarre avant l'étape (i)(a).

10. Procédé selon l'une quelconque des revendications 1 à 7, dans lequel la mise en œuvre de l'étape (i) (b) démarre après l'étape (i)(a).

11. Procédé selon l'une quelconque des revendications précédentes, dans lequel l'unité de traitement comprend

• un premier dispositif de distillation destiné à séparer une quatrième phase gazeuse contenant du phosgène de la troisième phase liquide pour obtenir une quatrième phase liquide appauvrie en phosgène,
• un deuxième dispositif de distillation destiné à séparer une cinquième phase gazeuse contenant du solvant de la quatrième phase liquide pour obtenir une cinquième phase liquide appauvrie en phosgène et en solvant et
• un troisième dispositif de distillation destiné à recueillir l'isocyanate de la cinquième phase liquide.

12. Procédé selon l'une quelconque des revendications précédentes, dans lequel, après avoir atteint l'état cible, la réaction continue de la solution d'amine avec la solution de phosgène est interrompue, les étapes suivantes pour l'interrompre étant réalisées :

(v) hausse de la température de la solution de phosgène et de la température de la solution d'amine à des débits de solution de phosgène et de solution d'amine inchangés par rapport à l'état cible ;
(vi) lorsqu'on atteint une température du mélange réactionnel sortant du troisième dispositif de mélange située dans la plage > 135 °C à 140 °C : coupure de l'alimentation en amine dans le premier dispositif de mélange tout en maintenant l'alimentation en solvant dans le premier dispositif de mélange et tout en maintenant l'alimentation en solution de phosgène dans le deuxième dispositif de mélange ;
(vii) coupure de l'alimentation en phosgène dans le deuxième dispositif de mélange tout en maintenant l'alimentation en solvant dans le deuxième dispositif de mélange et tout en maintenant l'alimentation en solvant dans le premier dispositif de mélange ;
(viii) après passage de la concentration de phosgène en dessous d'une limite prédéfinie dans le solvant sortant du deuxième dispositif de mélange : coupure de l'alimentation en solvant dans le deuxième dispositif de mélange ; et
(viii) coupure de l'alimentation en solvant dans le premier dispositif de mélange.

13. Procédé selon la revendication 12, dans lequel à l'étape (v), la température de la solution de phosgène est élevée à une valeur située dans la plage de 100 °C à 125 °C, et dans lequel la température de la solution d'amine est élevée à une valeur située dans la plage de 100 °C à 125 °C.

14. Procédé selon la revendication 12 ou 13, dans lequel à l'étape (vi), l'alimentation en amine est coupée dès que la température du mélange réactionnel sortant du troisième dispositif de mélange se situe dans la plage > 135 °C à 137 °C.

15. Procédé selon l'une quelconque des revendications précédentes, dans lequel l'amine primaire est choisie parmi la méthylène-diphénylène-diamine, la polyméthylène-polyphénylène-polyamine, un mélange de méthylène-diphénylène-diamine et de polyméthylène-polyphénylène-polyamine, la toluènediamine, la xylylènediamine, la 1,5-pentanediamine, la 1,6-hexaméthylènediamine, l'isophoronediamine et la naphtyldiamine.

**IN DER BESCHREIBUNG AUFGEFÜHRTE DOKUMENTE**

**In der Beschreibung aufgeführte Patentdokumente**

- DE 3403204 A
- DE 1768439 A
- DE 10222968 A
- EP 1873142 A1
- EP 1616857 A1
- EP 3653605 A1
- EP 3653604 A1
- WO 2015144658 A1
- EP 1854783 A2
- EP 0830894 A1
- EP 2077150 A1
- WO 2017050776 A1